# EUROPEAN PATENT APPLICATION

(11) **EP 4 647 081 A1**
(43) Date of publication of application: **12.11.2025**
(21) Application number: 24174521.5
(22) Date of filing: 07.05.2024
(51) Int. Cl.: A61K 38/00, A61K 39/00, A61P 37/00, C07K 14/415, C07K 14/315, C07K 14/31

(54) **CELL SURFACE IG-AIMED IMMUNE MEMORY ERASERS AND USES THEREOF**

(71) Applicant: Arakawa, Hiroshi, 20146 Milano (IT)
(72) Inventor: Arakawa, Hiroshi, 20146 Milano (IT)
(74) Representative: Pace Napoleone, Maria

(57) **Abstract**

The present invention refers to a fusion protein or conjugate comprising or consisting of:
a) a Diphtheria toxin deprived of the receptor binding domain or functional fragments or derivatives or a biologically active variant thereof; and
b) at least one surface immunoglobulin (slg) B-cell targeting peptide or functional fragments or derivatives or a biologically active variant thereof.

Isolated nucleic acids, vectors and pharmaceutical compositions related to said fusion protein, as well as their medical uses, are also objects of the invention.

## Description

### FIELD OF THE INVENTION

The present invention refers to fusion proteins or conjugates, herein named cell surface Ig-Aimed immune memory erasers (Ig-Aim), compositions and uses thereof, in particular for use for treating diseases related to disfunctions of B-cells, such as autoimmune diseases, allergies, B-cell dependent leukemia.

### BACKGROUND OF THE INVENTION

Autoimmune diseases are caused by the misdirection of the immune system as the side effect of antibody diversity, wherein each B cell possesses unique characteristics defined by its antibodies.

Antibodies possess remarkable diversity, enabling them to bind to a nearly limitless range of antigens. This variety of antibodies depends on complex mechanisms that edit the genome of individual B cells (1). The variable regions of antibodies are generated by V(D)J recombination and further modified by somatic hypermutation, resulting in increased affinity following exposure to antigens. In addition to the diversity in the variable regions, there are five different classes of constant regions in human antibodies. The classes, excluding IgM and IgD, are the outcome of switch recombination. Alongside gene conversion (*2*), switch recombination and somatic mutation (*3*) are initiated by Activation-Induced Cytidine Deaminase (AID).

Among the antibody classes, IgM is the first to be expressed during B cell development. Among them, the naive B cells have yet to encounter an antigen and represent the majority of the total B cell repertoire (*1*). However, in circulation in the blood, IgG is both the most abundant and has the longest serum half-life of all of the immunoglobulin classes (*1*). IgA plays a crucial role in protecting mucosal surfaces from viruses and bacteria (*1*). The least prevalent class, IgE, deals with parasitic infections but is also associated with allergies and anaphylaxis (*1*).

The B cell populations play a crucial role in acquired immunity, which primarily functions to combat foreign pathogens. However, B cells can also underly a number of pathologies, including allergy, autoimmune disease, and cancer (*1*). The misrecognition of autoantigens by B cells can lead to autoimmune diseases. Similarly, B cells activated by harmless substances like food or pollen can trigger allergies. Like many other cell types, B cells can develop tumors. Treatments for these B cell maladies often involve targeting the entire B-cell population in a nonspecific manner (*4*). This broad therapeutic approach can result in the elimination of large numbers of healthy, normal B cell populations along with the pathological population. Notably, the pathogenic B cells implicated in these various diseases often originate from just a single B cell that expresses a unique immunoglobulin which can be considered a stable biological marker for that specific B cell population. For instance, B-cell cancer drugs target markers common to B cells and do not distinguish between pathogenic and nonpathogenic cells. Thus, they can also kill nonpathogenic B cells, including naive B cells. Serum IgM constitutes about 10% of serum immunoglobulins in adults. Although IgM-class naive B cells are a minority of the cell population, they represent the repository of the B cell repertoire, estimated to over a million different types. Consequently, the loss of the naive B cells leads to B-cell immune deficiency.

Thus, it is highly desired an easily tunable therapeutic approach to specifically target a B cell expressing unique cell surface immunoglobulin (slg) in order to provide a safe alternative to conventional therapies and allow for the elimination of only pathogenic B cell populations.

Canonically, B cells produce immunoglobulins that are then secreted and proceed to opsonize antigens they encounter. However, B cells can also employ endocytosis when exposed to an antigen. This can occur when a membrane-bound immunoglobulin on the B cell's surface is exposed to a specific antigen. The antibody-antigen complex is then internalized and degraded. This process provides fragments of antigen that the B cell can then load on class II MHCs and present to helper T cells (*1*).

While membrane-bound and secreted immunoglobulins are produced by alternative splicing of the same immunoglobulin genes, slg⁺ B cells express membrane-bound antibodies on their cell surface as receptors to recognize specific antigens.

At the moment there are no therapeutic agents that exploit these unique features of B cell biology herein described, by targeting pathogenic B cells separately from nonpathogenic cells. Therefore, there is still the need of a therapeutic approach to specifically target and eliminate only pathogenic B cells for the treatment of autoimmune diseases and other B-cell diseases, avoiding the induction of general immunodeficiency.

The Diphtheria toxin is naturally produced by *Corynebacterium diphtheria.* The A fragment (N-terminal) of diphtheria toxin (DtA) contains a catalytic domain that disrupts protein synthesis in eukaryotic cells resulting in cytotoxicity in susceptible cells (*4*). The B fragment of the diphtheria toxin (C-terminus) consists of a transmembrane domain and a receptor-binding domain that facilitates the translocation of the catalytic domain from the endosome to the cytoplasm. As diphtheria toxin is not toxic to mice that naturally do not express its receptor, its toxicity requires binding to the specific receptor (*5*). By replacing the receptor-binding domain of diphtheria toxin with cytokines, growth factors, cancer-specific cell-penetrating peptides, and cancer cell antigens, this toxin has already been exploited as a therapeutic agent (*4*).

Staphylococcal protein A (SpA) (*6*) and Streptococcal protein G (SpG) (*7*) are commonly used molecules for binding antibodies, for example, in immunoprecipitation, enzyme-linked immunosorbent assay (ELISA), and Western blotting. SpA binds to immune complexes and serum immunoglobulins, making it widely utilized in pharmaceutical applications (*6*). Additionally, SpA can directly bind to antibodies on B cells (*6*). It has been reported that diphtheria toxin C-terminus fused with the SpA ZZ domain (*8*) or truncated Pseudomonas exotoxin A linked to the SpA ZZ domain (9) exhibit mild toxicity to cells through binding to antibodies against cell surface antigens, such as anti-Vero antiserum against Vero cells (*8*), anti-MUC1 antibody against MUC1-expressing cell lines (T47D and MCF7), and anti-ErbB2 receptor antibody against ErbB2 receptor-expressing cell lines (SKBR3, A431, T47D, MCF7, MDA-MB231) (*9*). However, their toxicity to B cells remains unknown. In addition, diphtheria toxin C-terminus retains the original toxicity of diphtheria, thus hampering in vivo use of said artificial toxin.

### SUMMARY OF THE INVENTION

The present invention provides a strategy for selectively removing target B-cell populations by targeting slg⁺ B cells specifically, using fusion proteins or conjugates, and compositions comprising those fusion proteins or conjugates, herein also named cell surface Ig-Aimed immune memory erasers (Ig-Aim). In particular, unique features of B cell biology were combined with the cytotoxic effect of diphtheria toxin to target specific populations of B cells.

In the fusion protein of the invention (IgAim) the receptor binding domain of diphtheria toxin is replaced with at least one surface immunoglobulin (slg) B cell targeting peptide, such as SpA, SpG, affibodies, or other specific antigens for slg⁺ B cells.

It was found that so obtained fusion proteins targeting B-cells expressing IgG (IgAimG) or B-cells expressing IgA (IgAimA) selectively killed slgG⁺ and slgA⁺ B cells, respectively. The treatment in vitro with fusion proteins IgAimG and IgAimA killed between 90% and 99% of the target cell population without displaying toxicity towards other classes of B cells or non-target cell types. Moreover, it was found that a fusion protein according to the invention comprising as targeting peptide pollen allergen Phleum pratense (Phl) p7 (IgAimP7), exclusively kills B cells expressing an antibody against pollen allergen Phleum pratense (Phl) p7, therefore highlighting the specificity of the fusion protein of the invention.

The protein of the invention advantageously targets surface immunoglobulin positive (slg+) B-cell(s) in a specific manner thus enabling the specific elimination of the target B cells or of the target IgG, IgA or IgE B cell population, without exhibiting toxicity towards the non-target B-cells, in particular IgM B cells that constitute the naïve B cell repertoire.

Proteins targeting B-cell surface markers are known but they target and kill not only pathogenic B-cells but also healthy B-cells. Instead, inventors herein exploit B-cell antibody diversity and demonstrate that the fusion proteins of the invention kill specifically pathological B cells, minimizing the damage to healthy B-cells.

The protein of the invention can therefore be advantageously used as a therapeutic agent for treating autoimmune diseases as well as B leukemia and other pathologies wherein a dysfunction of B-cells is involved.

It is therefore an object of the invention a fusion protein or conjugate comprising or consisting of:
a) a Diphtheria toxin deprived of the receptor binding domain or functional fragments or derivatives or a biologically active variant thereof; and
b) at least one surface immunoglobulin (slg) B-cell targeting peptide or functional fragments or derivatives or a biologically active variant thereof.

Preferably, said surface immunoglobulin (slg) B-cell targeting peptide of b) is selected from the group consisting of: peptides targeting a B-cell surface immunoglobulin G, peptides targeting a B-cell surface immunoglobulin A, peptides targeting a B-cell surface immunoglobulin E, peptides targeting a B-cell surface immunoglobulin against an autoimmune antigen, and peptides targeting a B-cell surface immunoglobulin against DNA.

Preferably, said at least one surface immunoglobulin (slg) B-cell targeting peptide of b) comprises or consists of at least one peptide selected from the group consisting of:
i. at least one peptide derived from Staphylococcal protein A, preferably said peptide comprising or consisting of a sequence having at least 80% of identity with the sequence of ZZ domain or ZigA domain of Staphylococcal protein A,
ii. at least one peptide derived from Streptococcal protein G, preferably said peptide comprising or consisting of a sequence having at least 80% of identity with the sequence of IgG binding domain of Streptococcal protein G, and
iii. at least one peptide derived from Phleum pratense p7 domain, preferably said peptide comprising or consisting of a sequence having at least 80% of identity with the sequence of Phleum pratense p7 domain, preferably said peptide is selected from (i.) a peptide comprising or consisting of at least one Staphylococcal protein A ZZ domain, (ii.) a peptide comprising or consisting of at least one, preferably two, Staphylococcal protein A ZigA domains (ZigA-ZigA) and a peptide comprising or consisting of at least one Phleum pratense p7 domain.

It is also an object of the invention, the fusion protein or conjugate, further comprising a linker, said linker being preferably selected from the group of:
GGGS linker, Proline-rich linker, and (Thr-Gly-Ser)n linker wherein n is 1, 2, 3, 4, 5, 6, 7, 8, 9, 10, 11, 12, 13, 14, 15, 16, 17, 18, 19, or 20, said linker being more preferably GGGS linker; and/or further comprising at least one peptide tag, said peptide tag being preferably selected from the group of:
histidine tag, Glutathione S-transferase (GST) tag, Maltose-binding protein (MBP) tag, S tag, FLAG tag, and Hemagglutinin (HA) tag, more preferably being histidine tag.

It is also an object of the invention, the fusion protein or conjugate wherein:
a) said diphtheria toxin has at least 80% sequence identity with, or comprises or consists of a sequence of SEQ ID NO. 4; preferably it comprises or consists of a sequence of SEQ ID NO. 4, and/or
b) said linker has at least 80% sequence identity with, or comprises or consists of a sequence of SEQ ID NO. 5, preferably it comprises or consists of a sequence of SEQ ID NO. 5; and/or
c) said surface immunoglobulin (slg) B cell targeting peptide has at least 80% sequence identity with, or comprises or consists of a sequence selected from the group consisting of:
   i. a sequence of SEQ ID NO. 6,
   ii. a sequence of SEQ ID NO. 13, and
   iii. a sequence of SEQ ID NO. 16,
      and/or
d) said peptide tag has at least 80% sequence identity with, or comprises or consists of

a sequence of SEQ ID NO. 7, preferably it comprises or consists of a sequence of SEQ ID NO: 7,
preferably said fusion protein or conjugate comprises from N-terminus to C-terminus said components a), b), c) and d).

Preferably, the fusion protein or conjugate is being able to target at least one surface immunoglobulin positive (slg+) B cell, preferably IgG+B-cells or IgA+B-cells, or IgE+B-cells, or Phleum pratense p7 expressing B-cells, or B-cells expressing surface antibodies against DNA.

Preferably, the fusion protein or conjugate is comprising from N-terminus to C-terminus:
i. a Diphtheria toxin deprived of the receptor binding domain comprising or consisting of a sequence having at least 80% sequence identity with, or comprising or consisting of a sequence of SEQ ID NO: 4 or functional fragments or derivatives or a biologically active variant thereof; and
ii. a GGGS linker comprising or consisting of a sequence having at least 80% sequence identity with, or comprising or consisting of a sequence of SEQ ID NO: 5; and
iii. a Staphylococcal protein A ZZ domain comprising or consisting of a sequence having at least 80% sequence identity with, or comprising or consisting of a sequence of SEQ ID NO: 6 or functional fragments or derivatives or a biologically active variant thereof; and
iv. a histidine tag comprising or consisting of a sequence having at least 80% sequence identity with, or comprising or consisting of a sequence of SEQ ID NO: 7;
   or

i. a Diphtheria toxin deprived of the receptor binding domain comprising or consisting of a sequence having at least 80% sequence identity with, or comprising or consisting of a sequence of SEQ ID NO: 4 or functional fragments or derivatives or a biologically active variant thereof; and
ii. a GGGS linker comprising or consisting of a sequence having at least 80% sequence identity with, or comprising or consisting of a sequence of SEQ ID NO: 5; and
iii. two Staphylococcal protein A ZigA (ZigA-ZigA) domains comprising or consisting of a sequence having at least 80% sequence identity with, or comprising or consisting of a sequence of SEQ ID NO: 13 or functional fragments or derivatives or a biologically active variant thereof; and
iv. a histidine tag comprising or consisting of a sequence having at least 80% sequence identity with, or comprising or consisting of a sequence of SEQ ID NO: 7;
   or

i. a Diphtheria toxin deprived of the receptor binding domain comprising or consisting of a sequence having at least 80% sequence identity with, or comprising or consisting of a sequence of SEQ ID NO: 4 or functional fragments or derivatives or a biologically active variant thereof; and
ii. a GGGS linker comprising or consisting of a sequence having at least 80% sequence identity with, or comprising or consisting of a sequence of SEQ ID NO: 5; and
iii. a Phleum pratense p7 domain comprising or consisting of a sequence having at least 80% sequence identity with, or comprising or consisting of a sequence of SEQ ID NO: 16 or functional fragments or derivatives or a biologically active variant thereof; and
iv. a histidine tag comprising or consisting of a sequence having at least 80% sequence identity with, or comprising or consisting of a sequence of SEQ ID NO: 7.

According to the present invention, the fusion protein or conjugate is having at least 80% sequence identity, preferably at least 90% sequence identity, with, or comprising or consisting of a sequence of SEQ ID NO: 1 (MGADDVVDSSKSFVMENFSSYHGTKPGYVDSIQKGIQKPKSGTQGNYDDDWKGF YSTDNKYDAAGYSVDNENPLSGKAGGVVKVTYPGLTKVLALKVDNAETIKKELGLSL TEPLMEQVGTEEFIKRFGDGASRVVLSLPFAEGSSSVEYINNWEQAKALSVELEINF ETRGKRGQDAMYEYMAQACAGNRVRRSVGSSLSCINLDWDVIRDKTKTKIESLKE HGPIKNKMSESPNKTVSEEKAKQYLEEFHQTALEHPELSELKTVTGTNPVFAGANY AAWAVNVAQVIDSETADNLEKTTAALSILPGIGSVMGIADGAVHHNTEEIVAQSIALS SLMVAQAIPLVGELVDIGFAAYNFVESIINLFQVVHNSYNRPAYSPGHKTSSGGGSS GGGSAVDNKFNKEQQNAFYEILHLPNLNEEQRNAFIQSLKDDPSQSANLLAEAKKL NDAQAPKVDNKFNKEQQNAFYEILHLPNLNEEQRNAFIQSLKDDPSQSANLLAEAK KLNGAQAPKVDANSAGKSTLEHHHHHH), or of a sequence of SEQ ID NO: 2 (MGADDVVDSSKSFVMENFSSYHGTKPGYVDSIQKGIQKPKSGTQGNYDDDWKGF YSTDNKYDAAGYSVDNENPLSGKAGGVVKVTYPGLTKVLALKVDNAETIKKELGLSL TEPLMEQVGTEEFIKRFGDGASRVVLSLPFAEGSSSVEYINNWEQAKALSVELEINF ETRGKRGQDAMYEYMAQACAGNRVRRSVGSSLSCINLDWDVIRDKTKTKIESLKE HGPIKNKMSESPNKTVSEEKAKQYLEEFHQTALEHPELSELKTVTGTNPVFAGANY AAWAVNVAQVIDSETADNLEKTTAALSILPGIGSVMGIADGAVHHNTEEIVAQSIALS SLMVAQAIPLVGELVDIGFAAYNFVESIINLFQVVHNSYNRPAYSPGHKTSSGGGSS GGGSAVDNKFNKETIQASQEIRLLPNLNGRQKLAFIHSLLDDPSQSANLLAEAKKLN DAQAPKVDNKFNKETIQASQEIRLLPNLNGRQKLAFIHSLLDDPSQSANLLAEAKKLN GAQAPKVDANSAGKSTLEHHHHHH), or of a sequence of SEQ ID NO: 3 (MGADDVVDSSKSFVMENFSSYHGTKPGYVDSIQKGIQKPKSGTQGNYDDDWKGF YSTDNKYDAAGYSVDNENPLSGKAGGVVKVTYPGLTKVLALKVDNAETIKKELGLSL TEPLMEQVGTEEFIKRFGDGASRVVLSLPFAEGSSSVEYINNWEQAKALSVELEINF ETRGKRGQDAMYEYMAQACAGNRVRRSVGSSLSCINLDWDVIRDKTKTKIESLKE HGPIKNKMSESPNKTVSEEKAKQYLEEFHQTALEHPELSELKTVTGTNPVFAGANY AAWAVNVAQVIDSETADNLEKTTAALSILPGIGSVMGIADGAVHHNTEEIVAQSIALS SLMVAQAIPLVGELVDIGFAAYNFVESIINLFQVVHNSYNRPAYSPGHKTSSGGGSS GGGSAASMADDMERIFKRFDTNGDGKISLSELTDALRTLGSTSADEVQRMMAEIDT DGDGFIDFNEFISFCNANPGLMKDVAKVFVDLEHHHHHH) or functional fragments, equivalents, variants, mutants, derivatives, synthetics or recombinants functional analogues thereof.

Another object of the present invention is an isolated nucleic acid encoding the fusion protein or conjugate, preferably said nucleic acid having at least 80% sequence identity, preferably at least 90% sequence identity, with or comprising or consisting of a sequence of SEQ ID NO. 8, SEQ ID NO. 14 or SEQ ID NO. 17.

A further object of the present invention is a recombinant expression vector comprising the isolated nucleic acid, preferably said isolated nucleic acid being operably linked to at least one regulatory sequence capable of directing expression of a gene encoding said fusion protein or conjugate in a host, or a host cell comprising and/or expressing the fusion protein or conjugate, or the isolated nucleic acid or said recombinant expression vector comprising the isolated nucleic acid, said host cell preferably being selected from the group consisting of: fungal cells, bacterial cells, preferably gram negative bacteria such as Escherichia coli, gram-positive Bacilli such as B. subtilis, B. licheniformis, B. megaterium, B. amyloliquefaciens, B. pumilus, plant protoplasts, unicellular algae, actinomycetales such as Streptomyces sp., and yeasts, such as Saccharomyces cerevisiae, Pichia pastoris, Yarrowia lipolytica, most preferably Trichoderma reesei or Bacillus, most preferably T7 Express Competent Escherichia coli.

Another object of the present invention is a method of producing the fusion protein or conjugate comprising the steps of transforming a host cell with an expression vector encoding said fusion protein, culturing said host cell under conditions enabling expression of said fusion protein, and optionally recovering and purifying said fusion protein.

A further object of the present invention is a composition comprising the fusion protein or conjugate, or a pharmaceutical composition comprising the fusion protein or conjugate and at least one pharmaceutically acceptable carrier.

Another object of the present invention is the fusion protein or conjugate, the isolated nucleic acid, the recombinant expression vector or the host cell, or the pharmaceutical composition for use as a medicament.

A further object of the present invention is the fusion protein or conjugate, the isolated nucleic acid, the recombinant expression vector or the host cell, or the pharmaceutical composition for use in the treatment of a disease related to a disfunction or a misfunctioning of B-cells or of a sub-population of B-cells.

Another object of the present invention is the fusion protein or conjugate or the isolated nucleic acid or the recombinant expression vector or the host cell or the pharmaceutical composition for the use wherein said disease is selected from the group consisting of: autoimmune diseases, allergies, B-cell dependent leukemia, nephropathy, Henoch-Schönlein purpura, celiac disease, and dermatologic herpetiformis, preferably said autoimmune disease being an autoimmune disease of musculoskeletal system, digestive system, nervous system, endocrine system, or skin.

### DETAILED DESCRIPTION OF THE INVENTION

The protein of the invention has the ability to eliminate antigen-specific memory selectively. Notably, the use of fusion protein directed to B-cells expressing IgG or to B-cells expressing IgA does not harm IgM class of B-cells, thereby preserving the naive B-cell repertoire, which constitutes the majority of the B-cell repertoire.

The selective elimination of B-cells is advantageous for treating immune disorders without inducing general immunodeficiency.

Numerous B leukemia cells depend on signaling from these cell surface antibodies and express them on their cell surface (*17*). B leukemias expressing IgG or IgA classes could be specifically targeted using fusion proteins of the invention specifically directed to IgG or IgA. For example, IgA nephropathy is caused by antibodies of the IgA class. The exceptional efficacy and specificity of the protein of the invention make it superior to currently available therapeutic options for B cell tumors.

Antibodies do not always defend against infections; in some instances, their presence can even facilitate infection. The phenomena of antibody-dependent enhancement (ADE) (*18*) and original antigenic sin (OAS) (*19*) highlight the potential limitations and challenges associated with relying solely on antibodies for protection against infections. ADE occurs when the binding of a virus to suboptimal antibodies enhances viral entry into host cells and promotes replication (*18*). On the other hand, OAS indicates the immune system's tendency to cling to the memory based on a previous infection, which can limit its ability to respond to mutant strains effectively (*19*). The protein of the invention can be advantageously used for the treatment of ADE or OAS to help erase these negative immune memories and address these challenges.

Any of the approximately 20,000 human genes can potentially give rise to an autoantigen, and the protein may become inactivated or activated depending on the antibody binding. While currently, the number of human diseases categorized as autoimmune diseases is limited (*20*), theoretically, there can be far more than 100 types of autoimmune diseases. By fusing the autoantigen with a portion of the diphtheria toxin according to the protein of the invention, specific personalized drugs for each autoimmune disease or allergy can be developed in order to eliminate antigen-specific memory by targeting the immune response.

The protein of the invention can advantageously mitigate the negative consequences associated with autoimmune diseases, specifically eliminate malignant B-cells, and provide a therapeutic strategy, for instance, for ADE.

The fusion protein of the invention can therefore be advantageously used for the treatment of any disease wherein a dysfunction or a misfunctioning of specific B-cells or of a sub-population of B-cells is involved. Such disease may be for example a disease related to pathogenic IgA, such as nephropathy, Henoch-Schönlein purpura, celiac disease, and dermatologic herpetiformis, or to pathogenic IgG, such as autoimmune diseases, or to pathogenic IgE, such as allergy and anaphylaxis.

Accordingly to the present invention, the fusion protein or the conjugate of the invention is able to target at least one surface immunoglobulin positive (slg+) B-cell. Preferably, it targets IgG+B-cells or IgA+B-cells, or IgE+B-cells or Phleum pratense p7 expressing B-cells, or B-cells expressing surface antibodies against DNA .

It is therefore an object of the present invention a fusion protein or conjugate comprising or consisting of:
a) a Diphtheria toxin deprived of the receptor binding domain or functional fragments or derivatives or a biologically active variant thereof; and
b) at least one surface immunoglobulin (slg) B-cell targeting peptide or functional fragments or derivatives or a biologically active variant thereof, said peptide being preferably selected from the group consisting of peptides targeting B-cell surface immunoglobulin G, peptides targeting B-cell surface immunoglobulin A, peptides targeting B-cell surface immunoglobulin E, peptides targeting B-cell surface immunoglobulins against an autoimmune antigen and peptides targeting B-cell surface immunoglobulins against DNA. In the context of the present invention for "a fusion protein or conjugate" is intended or may be defined as a product of recombinant DNA technology, obtained by genetically fusing at least two proteins, protein domains, or peptides together.

For "Diphtheria toxin deprived of the receptor binding domain" is intended a Diphtheria toxin which does not comprise or include the receptor-binding domain, which is known to be the domain that facilitates the translocation of the toxin catalytic domain from the endosome to the cytoplasm of the infected cell.

In an embodiment the Diphtheria toxin deprived of the receptor binding domain is an exotoxin consisting essentially of at least a portion of the A fragment of diphtheria toxin localized at N-terminus and at least a portion of the transmembrane domain of the B fragment of the diphtheria toxin localized at C-terminus. The A fragment of diphtheria toxin contains a catalytic domain that disrupts protein synthesis in eukaryotic cells exhibiting cytotoxic activity.

The Diphtheria toxin is secreted mainly by pathogenic bacterium that causes diphtheria, such as *Corynebacterium diphtheriae, Corynebacterium ulcerans* and *Corynebacterium pseudotuberculosis.* Sequence of the wild type Diphtheria toxin is known.

A Diphtheria toxin deprived of the receptor binding domain can be obtained according to common general knowledge in the field. In an embodiment, it is obtained by introducing amino acid substitutions of E82K and K179E into a vector coding for the inactive diphtheria toxin to revert it to the active form and by deleting amino acids 2-31 and 418-566, corresponding to the pel B signal peptide and the receptor binding domain.

According to the present invention, in the fusion protein of the invention the deprived receptor-binding domain localized at C-terminus of diphtheria toxin is replaced with at least one surface immunoglobulin (slg) B cell targeting peptide or functional fragments or derivatives or a biologically active variant thereof. Said peptide is preferably selected from the group consisting of peptides targeting B-cell surface immunoglobulin G, peptides targeting B-cell surface immunoglobulin A, peptides targeting B-cell surface immunoglobulin E, peptides targeting B-cell surface immunoglobulins against an autoimmune antigen and peptides targeting B-cell surface immunoglobulins against DNA. All such peptides can be obtained by the skilled person according to common general knowledge in the field. In particular, the sequences of B-cell surface immunoglobulins G, A and E are known in the art (*24*). B-cell surface immunoglobulins against an autoimmune antigen are also known, see for example the autoantigens in following Table 1.

**Table 1. List of autoimmune antigen(s) targeted by B-cell surface immunoglobulins**

| **Autoimmune Disease** | **Target Autoantigen(s)** |
|---|---|
| Systemic Lupus Erythematosus (SLE) | DNA, histones, Sm proteins, Ro/SSA, La/SSB |
| Rheumatoid Arthritis (RA) | Citrullinated protein antigens (CCP), Cartilage proteins |
| Sjögren's Syndrome (SS) | Ro/SSA, La/SSB, Alpha-fodrin |
| Hashimoto's Thyroiditis | Thyroid peroxidase (TPO) , Thyroglobulin (Tg), Thyroid stimulating hormone receptor (TSHR) |
| Graves' Disease | TSH receptor (TSHR), thyroglobulin |
| Type 1 Diabetes Mellitus (T1D) | Insulin-producing beta cells in the pancreas |
| Myasthenia Gravis (MG) | Acetylcholine receptors (AChRs) |
| Crohn's Disease | Cytoplasmic proteins of colon epithelial cells |
| Addison's Disease | Adrenal cortex cells |
| Celiac Disease | Complex of tissue transglutaminase and deamidated gluten peptide |
| Multiple Sclerosis | Myelin sheath |
| Scleroderma | Ro/SSA, centromere, topoisomerase I, RNA polymerase III |

In the context of the present invention "autoimmune antigen" and "autoantigen" are synonymous.

For "peptide targeting" or "targeting peptide" is intended a peptide able to bind to the target, e.g. the immunoglobulin, in particular to at least one target. The binding can be with any affinity, such as with low affinity or with high affinity.

For "surface immunoglobulin (slg) B-cell targeting peptide" is intended a peptide targeting an immunoglobulin located on the surface of a B-cell. "B-cell surface immunoglobulin (slg) targeting peptide" is a synonym.

For "B-cell surface immunoglobulins against DNA" is intended B-cell surface immunoglobulin able to bind to single-stranded (ss) or double-stranded (ds) DNA. Sequences of such immunoglobulins are also known (25).

In a preferred embodiment of the invention, said at least one surface immunoglobulin (slg) B-cell targeting peptide comprises or consists of at least one peptide selected from the group consisting of:
i. at least one peptide derived from Staphylococcal protein A, preferably comprising or consisting of a sequence having at least 80% of identity with the sequence of ZZ domain or ZigA domain of Staphylococcal protein A,
ii. at least one peptide derived from Streptococcal protein G, preferably comprising or consisting of a sequence having at least 80% of identity with the sequence of the IgG binding domain of Streptococcal protein G,
iii. at least one peptide derived from Phleum pratense p7 domain, preferably comprising or consisting of a sequence having at least 80% of identity with the sequence of Phleum pratense p7 domain.

Preferably, said peptide is selected from (i.) a peptide comprising or consisting of at least one Staphylococcal protein A ZZ domain, (ii.) a peptide comprising or consisting of at least one, preferably two, Staphylococcal protein A ZigA domains (ZigA-ZigA) and a peptide comprising or consisting of at least one Phleum pratense p7 domain. In an embodiment a peptide comprising or consisting of at least one Staphylococcal protein A ZZ domain of (i.) binds to IgG expressed on the surface of B-cells, a peptide comprising or consisting of at least one, preferably two, Staphylococcal protein A ZigA domains (ZigA-ZigA) of (ii.) binds to IgA expressed on the surface of B-cells and a peptide comprising or consisting of at least one Phleum pratense p7 domain (iii.) binds to p7 targeting IgG1 expressed on the surface of B-cells.

Sequences of Staphylococcal protein A, Streptococcal protein G and Phleum pratense, as well as respective domains, are all known in the art. For "peptide derived from" is intended a peptide comprising or consisting of a portion of the respective protein, for example the peptide comprises one or more domains of said protein or it is a fragment of said protein.

In exemplary embodiments, Staphylococcal protein A ZZ domain may be obtained from pKK-TEV-ProteinA (*22*), Streptococcal protein G may consist of amino acids 17-73 of pET His6 ProteinG TEV LIC cloning vector (1P), Staphylococcal protein A ZigA may be obtained by mutations introduced in Staphylococcal protein A sequence according to Gunneriusson et al. (*13*), Phleum pratense p7 domain consists of or comprises a full-length coding sequence of 78 amino acids (Y17835) of Phleum pratense p7.

In an embodiment 13 amino acidic surface residues of Staphylococcal protein A may be replaced to change its binding specificity to different target proteins (*12*). Such replacements may generate affibodies with altered binding specificities that mimic antibodies' binding properties.

Antibodies against DNA are known, see for example (*25*).

In an embodiment said surface immunoglobulin (slg) B-cell targeting peptide is cloned between the Diphtheria toxin domain and the histidine-tag (His-tag), when present, of an expression vector.

In an embodiment, said surface immunoglobulin (slg) B-cell targeting peptide can also be further selected from the group consisting of:
(a) peptides targeting B-cell surface immunoglobulin E (IgE), such as for example an IgE+ specific affibody.

For "affibody" is intended an engineered protein or peptide able to bind to a large number of target proteins or peptides with high affinity, imitating monoclonal antibodies. Affibodies can be obtained by modifying known antibodies or peptides or proteins known to bind to a specific target in order to render them able to bind to a different specific target. In an embodiment of the invention, said affibody is obtained from above mentioned proteins Staphylococcal protein A, or Streptococcal protein G or domains thereof. See for example (*13*).

The derived fusion protein (IgAimE) is able to kill specifically IgE (+) B-cells and therefore can be advantageously used as a therapeutic agent for the treatment of an allergy.

### (b) peptides targeting B-cell surface immunoglobulins against DNA.

Advantageously, according to this embodiment a fusion protein comprising said peptide is specific for B-cells comprising surface immunoglobulins against DNA regardless of their Ig class. It may be used as a therapeutic agent for the treatment of any disease characterized by the abnormal presence of B-cells directed against DNA, such as systemic lupus erythematosus (SLE), systemic sclerosis (SSc), Sjögren's syndrome, rheumatoid arthritis (RA) and mixed connective tissue disease (MCTD).

In a particular embodiment, said fusion protein further comprises one or more DNA sequences which are able to bind to B-cells comprising surface immunoglobulins against DNA. Such fusion proteins comprising DNA sequences may be obtained in vitro by binding biotin-labeled oligos or biotin-labeled truncated genomic DNA fragments with a fusion protein according to the invention conjugated with avidin.

### (c) peptides targeting B-cell surface immunoglobulins against autoantigens

For autoantigen is intended an antigen that is a normal bodily constituent and against which the immune system produces autoantibodies. Peptides targeting B-cell surface immunoglobulins against autoantigens can be designed according to common general knowledge. Typically they comprise at least a portion of an autoantigen, such as tripartite motif-containing protein 21 (TRIM21), an autoantigen in Sjögren's syndrome (26).

In one embodiment, said fusion protein targeting B-cell surface immunoglobulins against autoantigens can be obtained in vitro by binding the biotinylated peptide or protein of interest, such as an autoantigen, with a fusion protein according to the invention conjugated with avidin, to develop a personalized therapeutic agent for any autoimmune disease.

In an embodiment, such peptides can effectively target all IgG subpopulations, including IgG1, IgG2, IgG3, IgG4. Preferably, said peptides have higher killing efficiency against IgG (+) cells. Said peptides preferably comprise or consist of four repeats of the IgG-binding domain of Streptococcal protein G (SpG).

In the context of the present invention, "a cell surface immunoglobulin (slg) B cell targeting peptide" is intended or may be defined as a peptide specifically targeting a membrane-bound immunoglobulin expressed on B cell's surface.

It is a further object of the invention said fusion protein or conjugate further comprising a linker. In an embodiment a linker is being preferably selected from the group of:
i. GGGS linker, ii. Proline-rich linker, and iii. (Thr-Gly-Ser)n linker, wherein n is 1, 2, 3, 4, 5, 6, 7, 8, 9, 10, 11, 12, 13, 14, 15, 16, 17, 18, 19, or 20.

In a preferred embodiment said linker is GGGS linker.

In the context of the present invention, "linker" is intended or may be defined as a peptide linking at least two proteins, protein domains, or peptides together.

In an embodiment said linker is inserted between the diphtheria toxin deprived of the receptor binding domain of a) and the surface immunoglobulin (slg) B-cell targeting peptide or functional fragments or derivatives or a biologically active variant thereof of b).

In an embodiment said linker is attached to the C-terminal of the diphtheria toxin deprived of the receptor binding domain of a) and to the N-terminal of surface immunoglobulin (slg) B-cell targeting peptide or functional fragments or derivatives or a biologically active variant thereof of b).

In an embodiment said linker is inserted between the diphtheria toxin deprived of the receptor binding domain of a) and any one of peptides selected from (i.) Staphylococcal protein A ZZ domain, (ii.) two Staphylococcal protein A ZigA domains (ZigA-ZigA). (iii) Phleum pratense p7 domain.

In an embodiment said linker is attached to the C-terminal of the diphtheria toxin deprived of the receptor binding domain of a) and the N-terminal of any one of peptide selected from (i.) Staphylococcal protein A ZZ domain, (ii.) two Staphylococcal protein A ZigA domains (ZigA-ZigA). (iii) Phleum pratense p7 domain.

It is also an object of the invention said fusion protein further comprising at least one peptide tag, said peptide tag being preferably selected from the group of:
histidine tag, Glutathione S-transferase (GST) tag, Maltose-binding protein (MBP) tag, S tag, FLAG tag, and Hemagglutinin (HA) tag.

In the context of the present invention, for "S tag" is intended or may be defined as an oligopeptide derived from pancreatic ribonuclease A (RNase A) digested with subtilisin.

In the context of the present invention, for "FLAG tag" is intended or may be defined as an artificial peptide tag having the sequence of DYKDDDDK (SEQ ID NO. 26).

Preferably, said peptide tag is histidine tag.

In the context of the present invention, "protein tag" is intended or may be defined as a peptide sequence attached to fusion proteins or conjugates. It can be attached at either C-terminus or N-terminus or both C-terminus and N-terminus. Preferably, it is at C-terminus.

In the context of the present invention "protein tag" and "peptide tag" are the synonyms.

In one embodiment said peptide tag allows for the purification of said fusion protein or conjugate.

It is also an object of the invention the fusion protein wherein:
a) said diphtheria toxin has at least 80% sequence identity with, or comprises or consists of a sequence of SEQ ID NO. 4; preferably it comprises or consists of a sequence of SEQ ID NO. 4, and/or
b) said linker has at least 80% sequence identity with, or comprises or consists of a sequence of SEQ ID NO. 5, preferably it comprises or consists of a sequence of SEQ ID NO. 5; and/or
c) said surface immunoglobulin (slg) B cell targeting peptide has at least 80% sequence identity with, or comprises or consists of a sequence selected from the group of:
   i. a sequence of SEQ ID NO. 6,
   ii. a sequence of SEQ ID NO. 13,
   iii. a sequence of SEQ ID NO. 16,
      and/or
d) said peptide tag has at least 80% sequence identity with, or comprises or consists of a sequence of SEQ ID NO. 7, preferably it comprises or consists of a sequence of SEQ ID NO: 7.

In a preferred embodiment, the fusion protein or conjugate comprises from N-terminus to C-terminus components a), b), c) and d), as above defined.

It is also an object of the invention the fusion protein or conjugate comprising from N-terminus to C-terminus:
i. a Diphtheria toxin deprived of the receptor binding domain comprising or consisting of a sequence having at least 80% sequence identity with, or comprising or consisting of a sequence of SEQ ID NO. 4 or functional fragments or derivatives or a biologically active variant thereof; and
ii. a GGGS linker comprising or consisting of a sequence having at least 80% sequence identity with, or comprising or consisting of a sequence of SEQ ID NO. 5; and
iii. a Staphylococcal protein A ZZ domain comprising or consisting of a sequence having at least 80% sequence identity with, or comprising or consisting of a sequence of SEQ ID NO. 6 or functional fragments or derivatives or a biologically active variant thereof; and
iv. a histidine tag comprising or consisting of a sequence having at least 80% sequence identity with, or comprising or consisting of a sequence of SEQ ID NO. 7;
   or

i. a Diphtheria toxin deprived of the receptor binding domain comprising or consisting of a sequence having at least 80% sequence identity with, or comprising or consisting of a sequence of SEQ ID NO. 4 or functional fragments or derivatives or a biologically active variant thereof; and
ii. a GGGS linker comprising or consisting of a sequence having at least 80% sequence identity with, or comprising or consisting of a sequence of SEQ ID NO. 5; and
iii. two Staphylococcal protein A ZigA (ZigA-ZigA) domains comprising or consisting of a sequence having at least 80% sequence identity with, or comprising or consisting of a sequence of SEQ ID NO. 13 or functional fragments or derivatives or a biologically active variant thereof; and
iv. a histidine tag comprising or consisting of a sequence having at least 80% sequence identity with, or comprising or consisting of a sequence of SEQ ID NO. 7;
   or

i. a Diphtheria toxin deprived of the receptor binding domain comprising or consisting of a sequence having at least 80% sequence identity with, or comprising or consisting of a sequence of SEQ ID NO. 4 or functional fragments or derivatives or a biologically active variant thereof; and
ii. a GGGS linker comprising or consisting of a sequence having at least 80% sequence identity with, or comprising or consisting of a sequence of SEQ ID NO. 5; and
iii. a Phleum pratense p7 domain comprising or consisting of a sequence having at least 80% sequence identity with, or comprising or consisting of a sequence of SEQ ID NO. 16 or functional fragments or derivatives or a biologically active variant thereof; and
iv. a histidine tag comprising or consisting of a sequence having at least 80% sequence identity with, or comprising or consisting of a sequence of SEQ ID NO. 7.

It is also an object of the invention the fusion protein or conjugate having at least 80% sequence identity, preferably at least 85, 90, 91, 92, 93, 94, 95, 96, 97, 98, 99 or 100% sequence identity with, or comprising or consisting of a sequence of SEQ ID NO: 1 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
 
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAVDNKFNKEQQNAFYEILHLPNLNEEQRNAFIQSLKDDPSQSANLLAEAKKL
NDAQAPKVDNKFNKEQQNAFYEILHLPNLNEEQRNAFIQSLKDDPSQSANLLAEAK
KLNGAQAPKVDANSAGKSTLEHHHHHH),
 or of a sequence of SEQ ID NO: 2 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAVDNKFNKETIQASQEIRLLPNLNGRQKLAFIHSLLDDPSQSANLLAEAKKLN
DAQAPKVDNKFNKETIQASQEIRLLPNLNGRQKLAFIHSLLDDPSQSANLLAEAKKLN
GAQAPKVDANSAGKSTLEHHHHHH),
 or of a sequence of SEQ ID NO: 3 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAASMADDMERIFKRFDTNGDGKISLSELTDALRTLGSTSADEVQRMMAEIDT
DGDGFIDFNEFISFCNANPGLMKDVAKVFVDLEHHHHHH),
 or functional fragments, equivalents, variants, mutants, derivatives, synthetics or recombinants functional analogues thereof.

It is also an object of the invention the fusion protein or conjugate comprising or consisting of SEQ ID NO: 1 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
 
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAVDNKFNKEQQNAFYEILHLPNLNEEQRNAFIQSLKDDPSQSANLLAEAKKL
NDAQAPKVDNKFNKEQQNAFYEILHLPNLNEEQRNAFIQSLKDDPSQSANLLAEAK
KLNGAQAPKVDANSAGKSTLEHHHHHH),
 or of SEQ ID NO: 2 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAVDNKFNKETIQASQEIRLLPNLNGRQKLAFIHSLLDDPSQSANLLAEAKKLN
DAQAPKVDNKFNKETIQASQEIRLLPNLNGRQKLAFIHSLLDDPSQSANLLAEAKKLN
GAQAPKVDANSAGKSTLEHHHHHH),
 or of SEQ ID NO: 3 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAASMADDMERIFKRFDTNGDGKISLSELTDALRTLGSTSADEVQRMMAEIDT
DGDGFIDFNEFISFCNANPGLMKDVAKVFVDLEHHHHHH),
 or functional fragments, equivalents, variants, mutants, derivatives, synthetics or recombinants functional analogues thereof.

It is also an object of the invention an isolated nucleic acid encoding the fusion protein or conjugate of the invention, or functional fragments, equivalents, variants, mutants, derivatives, synthetics or recombinants functional analogues thereof.

Preferably, said nucleic acid has at least 80% sequence identity with, or comprises or consists of a sequence of SEQ ID NO. 8 
(ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
GGTTCTCGCACTAAAAGTGGATAATGCCGAAACTATTAAGAAAGAGTTAGGTTTA
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA
GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACGTCTAGCGGAGGTGGCTCTAGCGGTGGAGGATCC
GCAGTGGACAACAAATTCAACAAAGAACAACAAAACGCGTTCTATGAGATCTTA
CATTTACCTAACTTAAACGAAGAACAACGAAACGCCTTCATCCAAAGTTTAAAAG
ATGACCCAAGCCAAAGCGCTAACCTTTTAGCAGAAGCTAAAAAGCTAAATGATG
CTCAGGCGCCGAAAGTAGACAACAAATTCAACAAAGAACAACAAAACGCGTTCT
ATGAGATCTTACATTTACCTAACTTAAACGAAGAACAACGAAACGCCTTCATCCA
AAGTTTAAAAGATGACCCAAGCCAAAGCGCTAACCTTTTAGCAGAAGCTAAAAA
GCTAAATGGTGCTCAGGCGCCGAAAGTAGACGCGAATTCCGCGGGGAAGTCAA
CCCTCGAGCACCACCACCACCACCACTGA)
 or of SEQ ID NO. 14 
(ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
 
GGTTCTCGCACTAAAAGTGGATAATGCCGAAACTATTAAGAAAGAGTTAGGTTTA
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA
GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACGTCTAGCGGAGGTGGCTCTAGCGGTGGAGGATCC
GCAGTGGACAACAAATTCAACAAAGAAACCATCCAGGCGTCCCAGGAGATCCG
TCTGTTACCTAACTTAAACGGTCGTCAAAAACTGGCCTTCATCCACAGTTTACTG
GATGACCCAAGCCAAAGCGCTAACCTTTTAGCAGAAGCTAAAAAGCTAAATGAT
GCTCAGGCGCCGAAAGTCGACAACAAATTCAACAAAGAAACGATTCAGGCGTCT
CAGGAGATCCGCCTGTTACCTAACTTAAACGGTCGCCAAAAACTGGCCTTCATC
CATAGTTTACTGGATGACCCAAGCCAAAGCGCTAACCTTTTAGCAGAAGCTAAA
AAGCTAAATGGTGCTCAGGCGCCGAAAGTAGACGCGAATTCCGCGGGGAAGTC
AACCCTCGAGCACCACCACCACCACCACTGA)
 or of SEQ ID NO. 17

(ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
GGTTCTCGCACTAAAAGTGGATAATGCCGAAACTATTAAGAAAGAGTTAGGTTTA
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA

GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACGTCTAGCGGAGGTGGCTCTAGCGGTGGAGGATCA
GCAGCTAGCATGGCGGACGACATGGAGAGGATCTTCAAGAGGTTCGACACGAA
CGGTGACGGGAAGATCTCTCTGTCGGAGCTGACGGACGCACTGCGGACGCTG
GGCTCGACGTCGGCGGACGAGGTGCAGCGCATGATGGCGGAGATCGACACCG
ACGGCGACGGCTTCATCGACTTCAACGAGTTCATCTCCTTCTGCAACGCCAACC
CTGGACTCATGAAGGACGTCGCCAAGGTCTTCGTCGACCTCGAGCACCACCAC
CACCACCACTGA)

It is also an object of the invention a recombinant expression vector comprising or expressing said isolated nucleic acid. Preferably said isolated nucleic acid is operably linked to at least one regulatory sequence capable of directing expression of a gene encoding said fusion protein in a host.

It is also an object of the invention a host cell comprising and/or expressing the fusion protein or conjugate, or the isolated nucleic acid or the recombinant expression vector. Said host cell is preferably being selected from the group consisting of:
fungal cells, bacterial cells, preferably gram negative bacteria such as Escherichia coli, gram-positive Bacilli such as B. subtilis, B. licheniformis, B. megaterium, B. amyloliquefaciens, B. pumilus, plant protoplasts, unicellular algae, actinomycetales such as Streptomyces sp., and yeasts, such as Saccharomyces cerevisiae, Pichia pastoris, Yarrowia lipolytica, most preferably Trichoderma reesei or Bacillus, most preferably T7 Express Competent Escherichia coli.

It is also an object of the invention a method of producing the fusion protein or conjugate comprising the steps of transforming a host cell with an expression vector encoding said fusion protein, culturing said host cell under conditions enabling expression of said fusion protein, and optionally recovering and purifying said fusion protein.

In an embodiment said expression vectors are transformed into a host cell, such as for example T7 Express Competent E. coli (High Efficiency) commercially available at NEB, C2566. Said host cell is incubated with Isopropyl β-D-1-thiogalactopyranoside (IPTG) at 37 °C to induce the expression of said fusion protein or conjugate. The purification of said fusion protein or conjugate can be achieved by any method known in the art. For example, said host cell is lysed by lysis reagent (e.g. NEBExpress E. coli Lysis Reagent), and tagged protein is purified by purification column (e.g. NEBExpress Ni Spin Column). Fusion protein concentration can be measured by any method known in the art, such as for example with Qubit Protein Assay Kit.

It is also an object of the invention a composition comprising the fusion protein or conjugate or a pharmaceutical composition comprising the fusion protein or conjugate and at least one pharmaceutically acceptable carrier.

It is also an object of the invention the fusion protein or conjugate, the isolated nucleic acid encoding the fusion protein or conjugate, the recombinant expression vector comprising the isolated nucleic acid, the host cell comprising and/or expressing the fusion protein or conjugate, and the pharmaceutical composition comprising the fusion protein or conjugate for use as a medicament.

It is also an object of the invention the fusion protein or conjugate, the isolated nucleic acid encoding the fusion protein, the recombinant expression vector, the host cell comprising and/or expressing the fusion protein or conjugate, and the pharmaceutical composition comprising the fusion protein or conjugate for use in the treatment of a disease related to a disfunction or a misfunctioning of B-cells or of a sub-population of B-cells is involved, said disease being preferably selected from the group consisting of: autoimmune diseases, allergies, B-cell dependent leukemia, nephropathy, Henoch-Schönlein purpura, celiac disease, and dermatologic herpetiformis, preferably an autoimmune disease of musculoskeletal system, digestive system, nervous system, endocrine system, or skin.

For disease related to a disfunction or a misfunctioning of B-cells or of a sub-population of B-cells is intended a disease wherein B-cells do not function properly, for example a disease wherein there is an altered or abnormal presence or an altered or abnormal activity of B-cells or of a sub-population thereof.

Such disease may be for example a disease related to an excess production of IgA from B-cells, such as nephropathy, Henoch-Schönlein purpura, celiac disease, and dermatologic herpetiformis, or a disease related to an excess production of IgG, such as autoimmune diseases.

Representative autoimmune diseases of musculoskeletal system are: rheumatoid arthritis (RA), systemic lupus erythematosus (lupus), scleroderma, Sjögren's syndrome, dermatomyositis, ankylosing spondylitis, psoriatic arthritis, reactive arthritis, polymyalgia rheumatica.

Representative autoimmune diseases of digestive system are: celiac disease, inflammatory bowel disease (IBD), Crohn's disease, ulcerative colitis, autoimmune hepatitis, primary sclerosing cholangitis.

Representative autoimmune diseases of nervous system are: multiple sclerosis (MS), Guillain-Barré syndrome, myasthenia gravis.

Representative autoimmune diseases of endocrine system are: type 1 diabetes, Hashimoto's thyroiditis, Graves' disease, Addison's disease.

Representative autoimmune diseases of skin are: vitiligo, alopecia areata, psoriasis.

Other autoimmune diseases are: Raynaud's disease, vasculitis, sarcoidosis, autoimmune hemolytic anemia, idiopathic thrombocytopenic purpura (ITP).

In some embodiments, the protein of the invention can also be advantageously used for the treatment of antibody-dependent enhancement (ADE) and/or original antigenic sin (OAS) which may occur after a microbial infection.

Further objects of the invention are a nucleic acid encoding for the fusion protein or conjugate or functional fragments, equivalents, variants, mutants, derivatives, synthetics or recombinants functional analogues thereof as above defined, a vector, preferably a plasmid or a viral vector, preferably for gene therapy containing the said nucleic acid, and a nanoparticle comprising the fusion protein or conjugate as above defined, preferably the protein is adsorbed on the surface of gold nanoparticles.

In the context of the present invention the fusion protein also includes functional fragments, equivalents, variants, mutants, derivatives, synthetics or recombinants functional analogues thereof or conjugates or recombinant proteins or functional fragments, equivalents, variants, mutants, derivatives, synthetics or recombinants functional analogues thereof. In another embodiment the conjugate or fusion protein is in the form of nucleic acid, a plasmid or a viral vector for gene therapy. In another embodiment the fusion protein or conjugate is in the form of a nanoparticle, e.g. adsorbed on the surface of gold nanoparticles.

In the context of the present invention the mentioned peptides, polypeptides or proteins are preferably in a mature form. The term "peptide" also refers to polypeptide or protein. The peptide preferably comprises at least 5 amino acids, preferably between 5 and 600 amino acids, preferably between 400 and 550 amino acids.

In the context of the present invention a linker provides spatial separation between the different domain of the fusion proteins or conjugates of the invention. The linker may be a flexible linker. Non-limiting examples of flexible linkers that may be used in the invention include:
- (Gly4Ser)2 (SEQ ID NO. 19: GGGGSGGGGS);
- (Gly4Ser)3 (SEQ ID NO. 20: GGGGSGGGGSGGGGS);
- (Gly4Ser)4 (SEQ ID NO. 21: GGGGS GGGGS GGGGS GGGGS);
- (Gly4Ser)5 (SEQ ID NO. 22: GGGGSGGGGSGGGGSGGGGSGGGGS);
- S GGGGS GGGGS GGGGS (SEQ ID NO. 23);
- GGGGSGGGGSGGGAS (SEQ ID NO. 24);
- SSGGGSSGGGS (SEQ ID NO. 5).

The linker is preferably between 2 or 3 and 10 amino acids in length. Preferably, the linker of the invention is SSGGGSSGGGS (SEQ ID NO. 5).

The present invention relates to a conjugate or fusion protein formed through genetic fusion or chemical coupling.

In the method for producing a conjugate or fusion protein according to the invention, the expression of the conjugate may be carried out in any expression system known to the expert in the art.

Examples of expression systems are prokaryotic systems, such as *Bacillus subtilis, E. coli, Bacillus megaterium, Lactoccos Lactis.* Examples of eukaryotic expression systems are yeast systems, as e.g. *Saccharomyces cerevisiae* and P. *Pastoris,* fungus systems, as e.g. *Aspergillus niger and oryzae,* insects systems, mammalian systems, as e.g. HEK293 and CHO, transgenic plants or animals. All the expression systems mentioned in the publication Gomes et al., Advances in Animal and Veterinary Sciences, June 2016, 4(7):346-356 are herein incorporated by reference.

A polynucleotide or nucleic acid described herein can be present in a vector. A vector is a replicating polynucleotide, such as a plasmid, phage, or cosmid, to which another polynucleotide may be attached so as to bring about the replication of the attached polynucleotide. Construction of vectors containing a polynucleotide of the invention employs standard ligation techniques known in the art. See, e.g., Sambrook et al, Molecular Cloning: A Laboratory Manual, Cold Spring Harbor Laboratory Press (1989). A vector can provide for further cloning (amplification of the polynucleotide), i.e., a cloning vector, or for expression of the polynucleotide, i.e., an expression vector. The term vector includes, but is not limited to, plasmid vectors, viral vectors, cosmid vectors, transposon vectors, and artificial chromosome vectors. Examples of viral vectors include, for instance, adenoviral vectors, adeno-associated viral vectors, lentiviral vectors, retroviral vectors, and herpes virus vectors. A vector may be replication-proficient or replication- deficient. A vector may result in integration into a cell's genomic DNA. Typically, a vector is capable of replication in a host cell, for instance a mammalian and/or a bacterial cell, such as E. coli.

Selection of a vector depends upon a variety of desired characteristics in the resulting construct, such as a selection marker, vector replication rate, use in gene transfer into cells of the gastrointestinal tract, and the like. Suitable host cells for cloning or expressing the vectors herein are prokaryotic or eukaryotic cells. Suitable eukaryotic cells include mammalian cells, such as murine cells and human cells. Suitable prokaryotic cells include eubacteria, such as gram- negative organisms, for example, E. coli.

An expression vector optionally includes regulatory sequences operably linked to the polynucleotide of the present invention. An example of a regulatory sequence is a promoter. A promoter may be inducible, repressible, or constitutive, and examples of each type are known in the art. A polynucleotide of the present invention may also include a transcription terminator. Suitable transcription terminators are known in the art.

Polynucleotides described herein can be produced in vitro or in vivo. For instance, methods for in vitro synthesis include, but are not limited to, chemical synthesis with a conventional DNA/RNA synthesizer. Commercial suppliers of synthetic polynucleotides and reagents for in vitro synthesis are known. Methods for in vitro synthesis also include, for instance, in vitro transcription using a circular or linear expression vector in a cell free system. Expression vectors can also be used to produce a polynucleotide of the present invention in a cell, and the polynucleotide may then be isolated from the cell.

Also provided are compositions including one or more polypeptides or polynucleotides described herein. Such compositions typically include a pharmaceutically acceptable carrier. As used herein "pharmaceutically acceptable carrier" includes, but is not limited to, saline, solvents, dispersion media, coatings, antibacterial and antifungal agents, isotonic and absorption delaying agents, and the like, compatible with pharmaceutical administration. Additional compounds can also be incorporated into the compositions.

A composition may be prepared by methods known in the art of pharmacy. In general, a composition can be formulated to be compatible with its intended route of administration. A formulation may be solid or liquid. Administration may be systemic or local. In some aspects local administration may have advantages for site-specific, targeted disease management. Local therapies may provide high, clinically effective concentrations directly to the treatment site, with less likelihood of causing systemic side effects.

In the context of the present invention the term "comprising" includes the terms "comprising" and "consisting".

Included in the present invention are also nucleic acid sequences and amino acid sequences derived from the sequences shown herein and below, e.g. functional fragments, mutants, derivatives, analogues, precursors, variants and sequences having a % of identity of at least 80% with the sequences disclosed herein, and homologues of the peptides, proteins, sequences described herein.

Preferably the fragments, mutants, derivatives, analogues, precursors, variants and sequences having a % of identity of at least 80% with the sequences disclosed herein, and homologues of the peptides, proteins, sequences described herein are functional and/or biologically active.

For at least 80% is intended at least 80%, 85%, 86%, 87%, 88%, 89%, 90%, 91%, 92%, 93%, 94%, 95%, 96%, 97%, 98%, 99% or 100%.

When describing the present invention, all terms not defined herein have their common art-recognized meanings. Any term or expression not expressly defined herein shall have its commonly accepted definition understood by those skilled in the art. To the extern that the following description is of a specific embodiment or a particular use of the invention, it is intended to be illustrative only, and not limiting of the claimed invention. The following description is intended to cover all alternatives, modifications and equivalents that are included in the spirit and scope of the invention, as defined in the appended claims.

The present invention also includes functional fragments, variants or derivatives of the proteins, peptides, conjugates or sequences herein disclosed. In the context of the present invention, when referring to specific DNA sequences, it is intended that it is comprised within the invention also RNA molecules identical to said polynucleotides, except for the fact that the RNA sequence contains uracil instead of thymine and the backbone of the RNA molecule contains ribose instead of deoxyribose, RNA sequence complementary the sequences therein disclosed, functional fragments, mutants and derivatives thereof, proteins encoded therefrom, functional fragments, mutants and derivatives thereof. The term "functional" may be understood as capable of maintaining the same activity of the reference peptide, protein, sequence. "Fragments" are preferably long at least 10 aa., 20 aa., 30 aa., 40 aa., 50 aa., 60 aa., 70 aa., 80 aa., 90 aa., 100 aa. "Derivatives" may be recombinant or synthetic. The term "derivative" as used herein in relation to a protein means a chemically modified protein or an analogue thereof, wherein at least one substituent is not present in the unmodified protein or an analogue thereof, i.e. a protein which has been covalently modified. Typical modifications are amides, carbohydrates, alkyl groups, acyl groups, esters and the like. As used herein, the term "derivatives" also refers to longer or shorter polynucleotides/proteins and/or having e.g. a percentage of identity of at least 70%, 75%, 80%, 85%, 86%, 87%, 88%, 89%, 90%, 91%, 92%, 93%, 94%, 95%, 96%, 97%, 98%, more preferably of at least 99% with the sequences herein disclosed. In the present invention "at least 70 % identity" means that the identity may be at least 70%, or 75%, or 80%, or 85 % or 90% or 95% or 100% sequence identity to referred sequences. This applies to all the mentioned % of identity. In the present invention "at least 80 % (sequence) identity" means that the identity may be at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to referred sequences. This applies to all the mentioned % of identity. Preferably, the % of identity relates to the full length of the referred sequence. The derivative of the invention also includes "functional mutants" of the polypeptides, which are polypeptides that may be generated by mutating one or more amino acids in their sequences and that maintain their activity. Indeed, the polypeptide of the invention, if required, can be modified in vitro and/or in vivo, for example by glycosylation, myristoylation, amidation, carboxylation or phosphorylation, and may be obtained, for example, by synthetic or recombinant techniques known in the art. Also within the scope of the subject invention are polynucleotides which have the same nucleotide sequences of a polynucleotide exemplified herein except for nucleotide substitutions, additions, or deletions within the sequence of the polynucleotide, as long as these variant polynucleotides retain substantially the same relevant functional activity as the polynucleotides specifically exemplified herein (e.g., they encode a protein having the same amino acid sequence or the same functional activity as encoded by the exemplified polynucleotide). Thus, the polynucleotides disclosed herein should be understood to include mutants, derivatives, variants and fragments, as discussed above, of the specifically exemplified sequences. The subject invention also contemplates those polynucleotide molecules having sequences which are sufficiently homologous with the polynucleotide sequences of the invention so as to permit hybridization with that sequence under standard stringent conditions and standard methods (Maniatis, T. et al, 1982). Polynucleotides described herein can also be defined in terms of more particular identity and/or similarity ranges with those exemplified herein. The sequence identity will typically be greater than 60%, preferably greater than 75%, more preferably greater than 80%, even more preferably greater than 90%, and can be greater than 95%. The identity and/or similarity of a sequence can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 , 92, 93, 94, 95, 96, 97, 98, or 99% or greater as compared to a sequence exemplified herein. Unless otherwise specified, as used herein percent sequence identity and/or similarity of two sequences can be determined e.g. using the algorithm of Karlin and Altschul (1990), modified as in Karlin and Altschul (1993). Such an algorithm is incorporated into the NBLAST and XBLAST programs of Altschul et al. (1990). BLAST searches can be performed with the NBLAST program, score = 100, word length = 12, to obtain sequences with the desired percent sequence identity. To obtain gapped alignments for comparison purposes, Gapped BLAST can be used as described in Altschul et al. (1997). When utilizing BLAST and Gapped BLAST programs, the default parameters of the respective programs (NBLAST and XBLAST) can be used. See NCBI/NIH website.

The peptide or the protein as above defined may include an amino acid sequence with at least 80%, 82 %, 85 %, 90 %, 92 %, 95%, 98%, 99% or 100% identity to the sequences herein mentioned. Determining percent identity of two amino acid sequences may include aligning and comparing the amino acid residues at corresponding positions in the two sequences. If all positions in two sequences are occupied by identical amino acid residues then the sequences are said to be 100% identical. Percent identity may be measured by the Smith Waterman algorithm (Smith T F, Waterman M S 1981 "Identification of Common Molecular Subsequences," J Mol Biol 147: 195-197, which is incorporated herein by reference as if fully set forth). The peptide and the protein herein disclosed may have fewer or more than the residues of the mentioned sequences or may present amino acid replacement in comparison to the herein mentioned sequences. The replacement may be with any amino acid whether naturally occurring or synthetic. The replacement may be with an amino acid analogue or amino acid mimetic that functions similarly to the naturally occurring amino acids. Naturally occurring amino acids are those encoded by the genetic code, as well as those amino acids that are later modified. The later modification may be but is not limited to hydroxyproline, γ-carboxyglutamate, and O-phosphoserine modifications. Naturally occurring amino acids include the standard 20, and unusual amino acids. Unusual amino acids include selenocysteine. The replacement may be with an amino acid analogue, which refers to compounds that have the same basic chemical structure as a naturally occurring amino acid; e.g., a carbon that is bound to a hydrogen, a carboxyl group, an amino group, and an R group. Examples of amino acid analogues include but are not limited to homoserine, norleucine, methionine sulfoxide, methionine methyl sulfonium. Such analogues may have modified R groups or modified peptide backbones. The amino acid analogues may retain the same basic chemical structure as a naturally occurring amino acid. The replacement may be with an amino acid mimetic, which refers to chemical compounds that have a structure that is different from the general chemical structure of an amino acid, but that functions similarly to a naturally occurring amino acid. The replacement may be with an α, α-disubstituted 5-carbon olefinic unnatural amino acid. A replacement may be a conservative replacement, or a non-conservative replacement. A conservative replacement refers to a substitution of an amino acid with a chemically similar amino acid. Conservative substitution tables providing functionally similar amino acids are well known in the art. Such conservatively replacements include but are not limited to substitutions for one another: (1) Alanine (A), Glycine (G); (2) Aspartic acid (D), Glutamic acid (E); (3) Asparagine (N), Glutamine (Q); (4) Arginine (R), Lysine (K); (5) Isoleucine (I), Leucine (L), Methionine (M), Valine (V); (6) Phenylalanine (F), Tyrosine (Y), Tryptophan (W); (7) Serine (S), Threonine (T); and (8) Cysteine (C), Methionine (M) (see, e.g., Creighton, Proteins (1984)). A replacement may be from one amino acid to another with a similar hydrophobicity, hydrophilicity, solubility, polarity, or acidity. A sequence having less than 100% identity to the mentioned sequences may be referred to as a variant. In an embodiment, one or more amino acids residues are replaced with a residue having a crosslinking moiety. As used herein, a "peptide" or "polypeptide" comprises a polymer of amino acid residues linked together by peptide (amide) bonds. The term(s), as used herein, refer to proteins, polypeptides, and peptide of any size, structure, or function. Typically, a peptide or polypeptide will be at least three amino acids long. A peptide or polypeptide may refer to an individual protein or a collection of proteins. The peptides of the instant invention may contain natural amino acids and/or non-natural amino acids (i.e., compounds that do not occur in nature but that can be incorporated into a polypeptide chain). Amino acid analogues as are known in the art may alternatively be employed. One or more of the amino acids in a peptide or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification. A peptide or polypeptide may also be a single molecule or may be a multimolecular complex, such as a protein. A peptide or polypeptide may be just a fragment of a naturally occurring protein or peptide. A peptide or polypeptide may be naturally occurring, recombinant, or synthetic, or any combination thereof. A large number of agents are developed to target cellular contents, cellular compartments, or specific protein, lipid, nucleic acid or other targets or biomarkers within cells. While these agents can bind to their intracellular targets with strong affinity, many of these compounds, whether they be molecules, proteins, nucleic acids, peptides, nanoparticles, or other intended therapeutic agents or diagnostic markers cannot cross the cell membrane efficiently or at all.

In the context of the present invention, the sequence having a certain % of identity with the reference sequences herein identified (e.g. having at least 80% sequence identity), or the functional fragments or derivatives or a biologically active variant of the reference sequences herein identified preferably present the same activity of the reference sequence.

The composition of the invention may include a pharmaceutically acceptable carrier. The pharmaceutically acceptable carrier may include but is not limited to at least one of ion exchangers, alumina, aluminum stearate, lecithin, serum proteins, human serum albumin, buffer substances, phosphates, glycine, sorbic acid, potassium sorbate, partial glyceride mixtures of saturated vegetable fatty acids, water, salts, electrolytes, protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, waxes, polyethylene glycol, starch, lactose, dicalcium phosphate, microcrystalline cellulose, sucrose, dextrose, talc, magnesium carbonate, kaolin; non-ionic surfactants, edible oils, physiological saline, bacteriostatic water, Cremophor EL^{™} (BASF, Parsippany, N.J.), and phosphate buffered saline (PBS).

Administering may include delivering a dose of 1 ng/kg/day to 100 µg/kg/day of the fusion protein or conjugate product. The dose may be any value between 1 ng/kg/day to 100 µg/kg/day. The dose may be any dose between and including any two integer values between 1 ng/kg/day to 100 µg/kg/day. The dose may be 10, 15, 20, 25, 30, 35, 40, 45, 50, 55, 60, 65, 70, 75, 80, 85, 90, 95, or 100 ng/kg/day or mg/kg/day or any dose in a range between any two of the foregoing. Preferably, the dose may be about 16 ng/kg/day. Administering may include delivering any dose of a complementing therapeutic. The complementing therapeutic dose may be any 1 to 100 mg/kg/day. The complementing therapeutic dose may be any value between 1 to 100 mg/kg/day. The complementing therapeutic dose may be any dose between and including any two integer values between 1 ng/kg/day to 100 mg/kg/day. The complementing therapeutic dose may be 10, 15, 20, 25, 30, 35, 40, 45, 50, 55, 60, 65, 70, 75, 80, 85, 90, 95, or 100 mg/kg/day or any dose in a range between any two of the foregoing. The complementing therapeutic may be any one or more of nanoparticle (e.g. gold nanoparticles, liposomes), a therapeutic agent (e.g. cytokines, chemotherapeutic drugs, antibodies and antibody fragments, toxins, nucleic acids), a diagnostic agent (e.g. radioactive compounds, fluorescence compounds, chemiluminescent compounds), a contrasting agent (e.g. microbubbles), or cellular components (e.g. chimeric antigen receptors or TCRs). The concentration of the peptide(s) and at least one complementing therapeutic in the composition may be set to deliver the daily (or weekly, every three weeks or monthly) dosage in a single administration, two-point administrations, multiple point administrations, or continuous administration (e.g., intravenously, transdermally, intraperitoneally, by isolated limb perfusion, by isolated hepatic perfusion, or local administration) over a period of time. The period may be one day. The period may be 1, 2, 4, 8, 12, or 24 hours or a time within a range between any two of these values. The fusion peptide or conjugate and complementing therapeutic can be administered simultaneously or with 1, 2, 4, 8, 12, 24, 48 hours of delay or anticipation or any intermediate times.

A composition including fusion protein or conjugate product of the invention may include any amount of the protein or product. The amount may be that sufficient to deliver the dosage as set forth above in a suitable volume or sized delivery mode. When the dosage is split into multiple administrations throughout a time period, the amount in one volume or delivery mode may be the total dosage divided by the number of administrations throughout the time period. When present in a composition, the complementing therapeutic may be at any complementing therapeutic amount. Like the peptide, the complementing therapeutic amount may be tailored to deliver the right complementing therapeutic amount in the volume or delivery mode used for administration.

The patient may be an animal. The patient may be a mammal. The patient may be a human. The patient may be patient affected by disease related to a disfunction of B-cells, preferably a disease selected from autoimmune diseases, allergies, B-cell dependent leukemia, nephropathy, Henoch-Schönlein purpura, celiac disease, and dermatologic herpetiformis, preferably an autoimmune disease of musculoskeletal system, digestive system, nervous system, endocrine system, or skin. The route for administering a composition or pharmaceutical composition may be by any route. The route of administration may be any one or more route including but not limited to oral, injection, topical, enteral, rectal, gastrointestinal, sublingual, sublabial, buccal, epidural, intracerebral, intracerebroventricular, intracisternal, epicutaneous, intraderm al, subcutaneous, nasal, intravenous, intraarterial, intramuscular, intracardiac, intraosseous, intrathecal, intraperitoneal, intravesical, intravitreal, intracavernous, intravaginal, intrauterine, extra-amniotic, transdermal, intratumoral, and transmucosal. Embodiments include a method of making the peptides of the invention, including the stapled peptide.

The method may include synthesizing a fusion protein or conjugate product having the sequence of the selected modified peptide.

An embodiment includes fusion protein or conjugate product or a composition thereof comprising a peptide consisting of, consisting essentially of, or comprising the sequence of any amino acid sequence herein. The peptide composition may include any complementing therapeutic herein. The peptide composition may include a pharmaceutically acceptable carrier.

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10 or 20 amino acids.

It will be understood that polypeptide sequences or peptides or proteins of the invention are not limited to the particular sequences or fragments thereof but also include homologous sequences obtained from any source, for example related viral bacterial proteins, cellular homologues and synthetic peptides, as well as variants or derivatives thereof. Polypeptide sequences of the present invention also include polypeptides encoded by polynucleotides of the present invention.

The terms "variant" or "derivative" in relation to the amino acid sequences of the present invention includes any substitution of, variation of, modification of, replacement of, deletion of or addition of one (or more) amino acids from or to the sequence providing the resultant amino acid sequence preferably has targeting activity, preferably having at least 25 to 50% of the activity as the polypeptides herein presented, more preferably at least substantially the same activity. Thus, sequences may be modified for use in the present invention. Typically, modifications are made that maintain the activity of the sequence. Thus, in one embodiment, amino acid substitutions may be made, for example from 1, 2 or 3 to 10, 20 or 30 substitutions provided that the modified sequence retains at least about 25 to 50% of, or substantially the same activity. However, in an alternative embodiment, modifications to the amino acid sequences of a polypeptide of the invention may be made intentionally to reduce the biological activity of the polypeptide. For example, truncated polypeptides that remain capable of binding to target molecule but lack functional effector domains may be useful. In general, preferably less than 20%, 10% or 5% of the amino acid residues of a variant or derivative are altered as compared with the corresponding region depicted in the sequence listings. Amino acid substitutions may include the use of non-naturally occurring analogues, for example to increase blood plasma half-life of a therapeutically administered polypeptide.

Polypeptides of the invention also include fragments of the above-mentioned polypeptides and variants thereof, including fragments of the sequences. Preferred fragments include those which include an epitope. Suitable fragments will be at least about 5, e.g. 10, 12, 15 or 20 amino acids in length. They may also be less than 200, 100 or 50 amino acids in length. Polypeptide fragments of the proteins and allelic and species variants thereof may contain one or more (e.g. 2, 3, 5, or 10) substitutions, deletions or insertions, including conserved substitutions. Where substitutions, deletion and/or insertions have been made, for example by means of recombinant technology, preferably less than 20%, 10% or 5% of the amino acid residues depicted in the sequence listings are altered. Proteins of the invention are typically made by recombinant means. However, they may also be made by synthetic means using techniques well known to skilled persons such as solid phase synthesis. Various techniques for chemical synthesizing peptides are reviewed by Borgia and Fields, 2000, TibTech 18: 243-251 and described in detail in the references contained therein.

Methods for preparing the conjugates of the invention have been described e.g. in WO01/61017.

The present invention will be described by means of non-limiting examples, referring to the following figures:

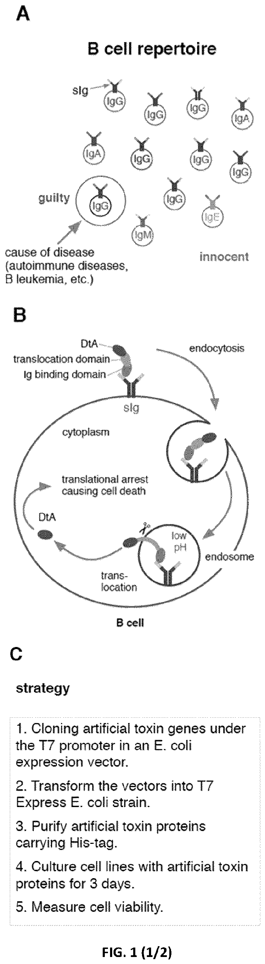

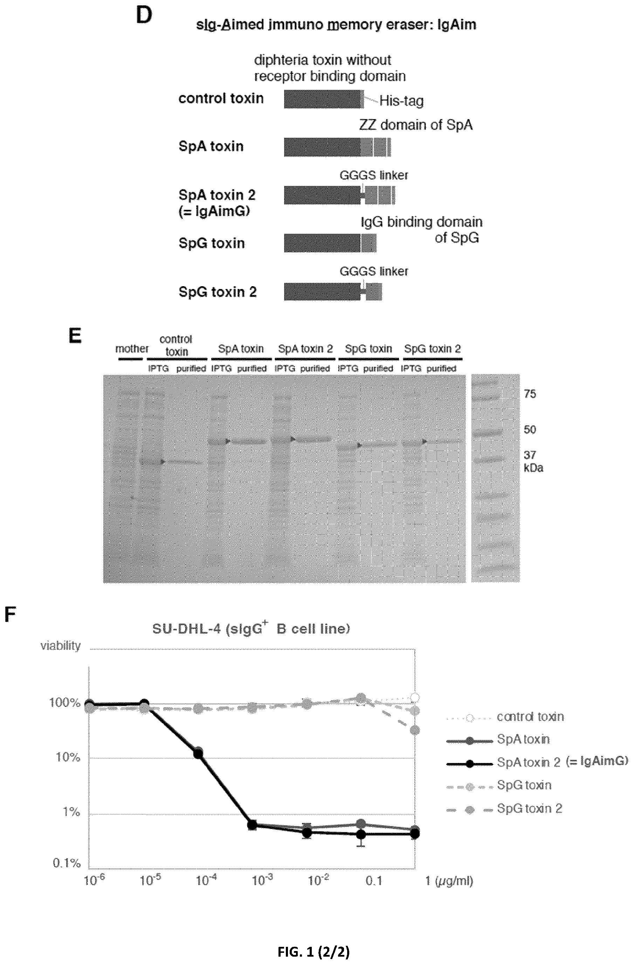

**Figure 1****. IgAim: cell surface Ig-Aimed Immune Memory erasers. A.** Illustration depicting the uniqueness of B cells. Cell surface Ig (slg) is a membrane-bound immunoglobulin, and its antibody class and antigen specificity can distinguish each slg⁺ B cell. **B.** Expected mechanism of action of the antibody-binding toxin. After binding to the antibody, the toxin is internalized into the cell by endocytosis and translocated into the cytoplasm, causing translation arrest and cell death. **C.** Overview of the strategy. **D.** Design of IgAim. One control toxin and four different toxins against slgG⁺ B cells. The SpA toxin 2 was renamed to IgAimG. **E.** Isopropyl β-D-1-thiogalactopyranoside (IPTG) induction and purification of IgAim proteins. Purified toxin proteins are highlighted by triangles. **F.** Evaluation of IgAim cytotoxicity against the slgG⁺ B cell line SU-DHL-4. Experiments were performed in triplicate.

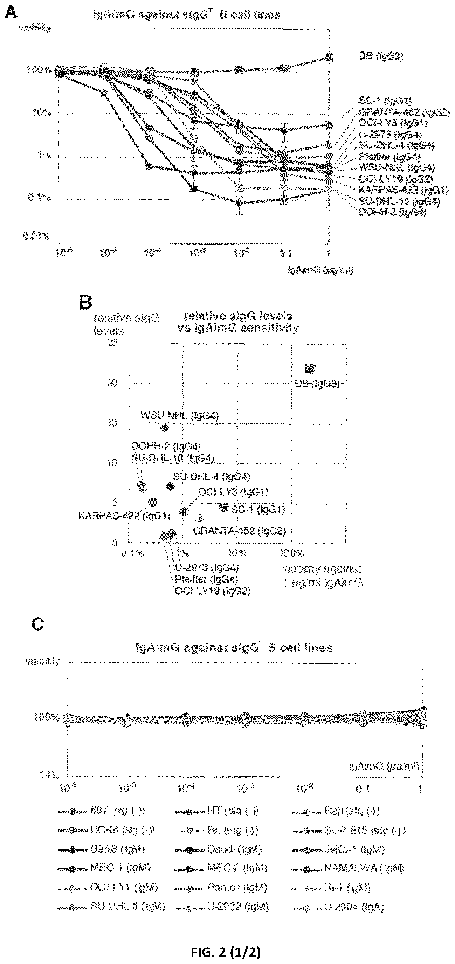

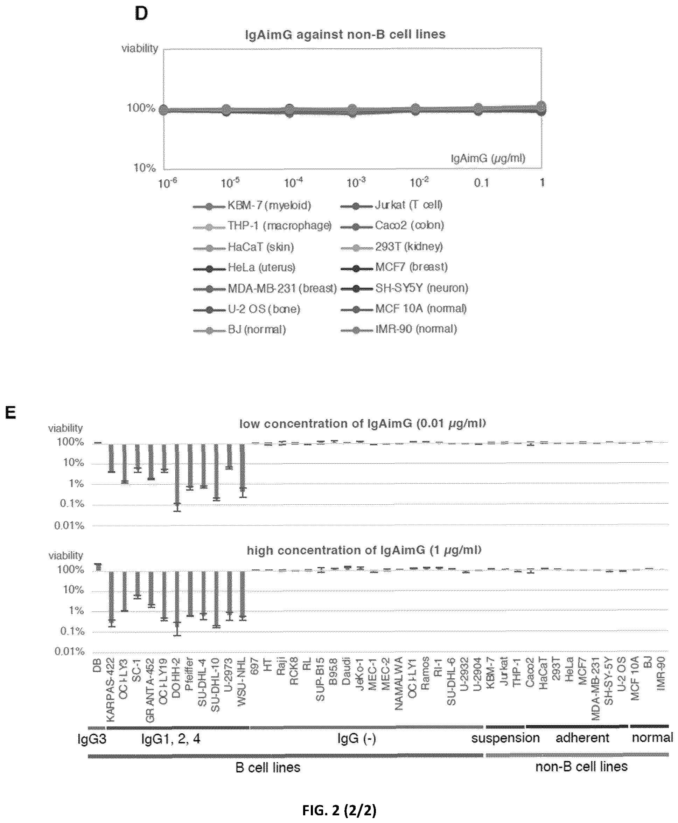

**Figure 2****. IgAimG: slgG⁺ B cell eraser. A.** The cytotoxicity of IgAimG to the slgG⁺ B cell lines. The IgG subclasses of the B cell lines are also indicated. Circles, triangles, squares, and diamonds represent the B cell subclasses, IgG1, IgG2, IgG3, and IgG4, respectively. **B.** IgG expression levels of slgG⁺ B cells were evaluated using flow cytometry and normalized. These slgG levels of cell lines are plotted against their viability in the presence of 1 µg/ml IgAimG in Fig. 2A. The symbols for each cell line are the same as in Fig. 2A. C. The cytotoxicity of IgAimG to the sIgG⁻ B cell lines. The Ig classes of the B cell lines are also indicated. **D.** The cytotoxicity of IgAimG to non-B cell lines. The origin or other characteristics of the cell lines are also indicated. **E.** Comparison of the cytotoxicity of high and low concentrations of IgAimG to B cell lines and other cell lines. Experiments in Fig. 2A, C, and D were performed in triplicate, although error bars were not shown in Fig. 2C and D.

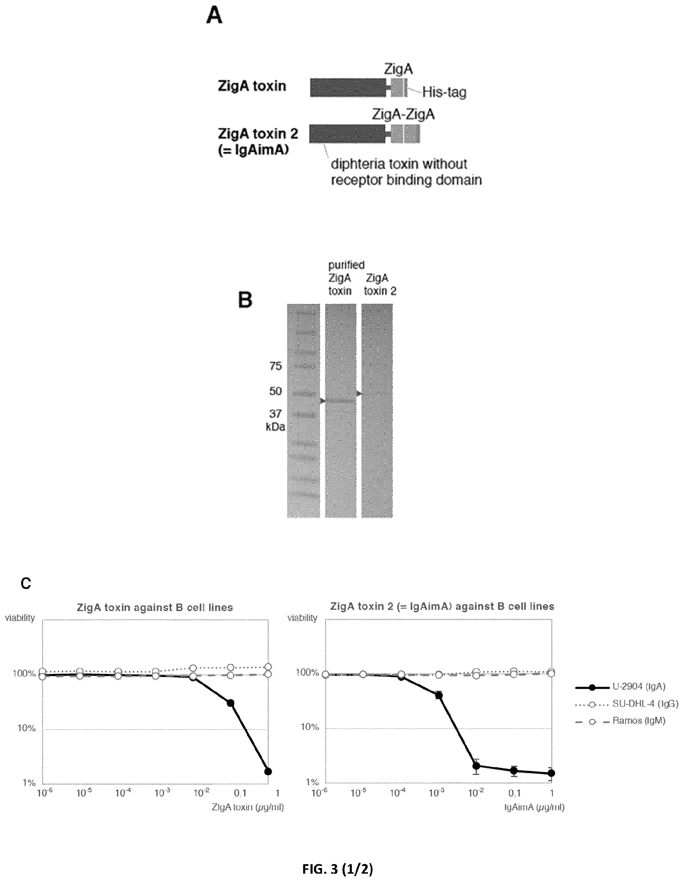

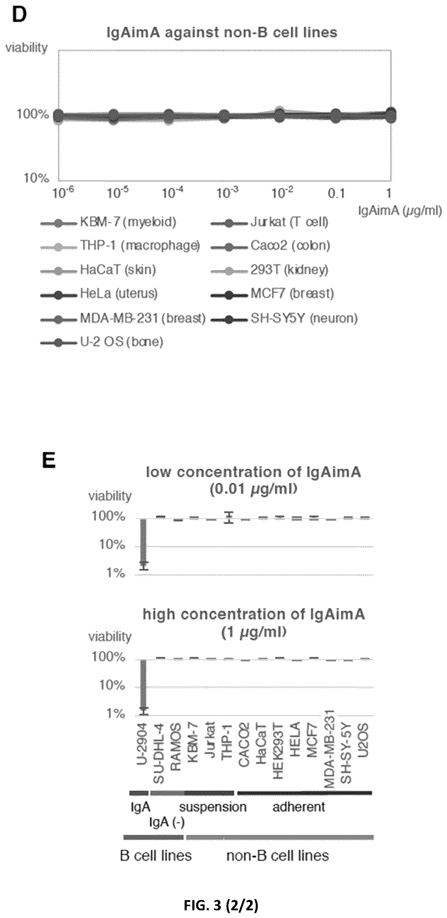

**Figure 3****. IgAimA: sIgA⁺ B cell eraser. A.** Two toxins using affibodies against IgA (ZigA). The ZigA toxin 2 was renamed to IgAimA. **B.** Purification of the toxin proteins. Purified toxin proteins are highlighted by triangles. **C.** The cytotoxicity of IgA toxins against the B cell lines. **D.** The cytotoxicity of IgAimA to non-B cell lines. Experiments in Fig. 3C and D were performed in triplicate, although error bars were not shown in Fig. 3D. **E.** Comparison of the cytotoxicity of high and low concentrations of IgAimA against B cell lines and other cell lines.

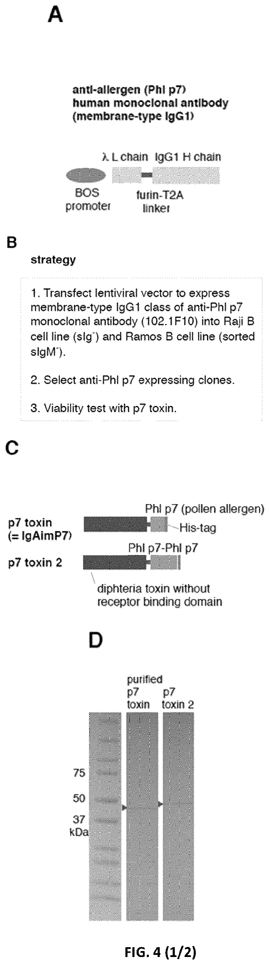

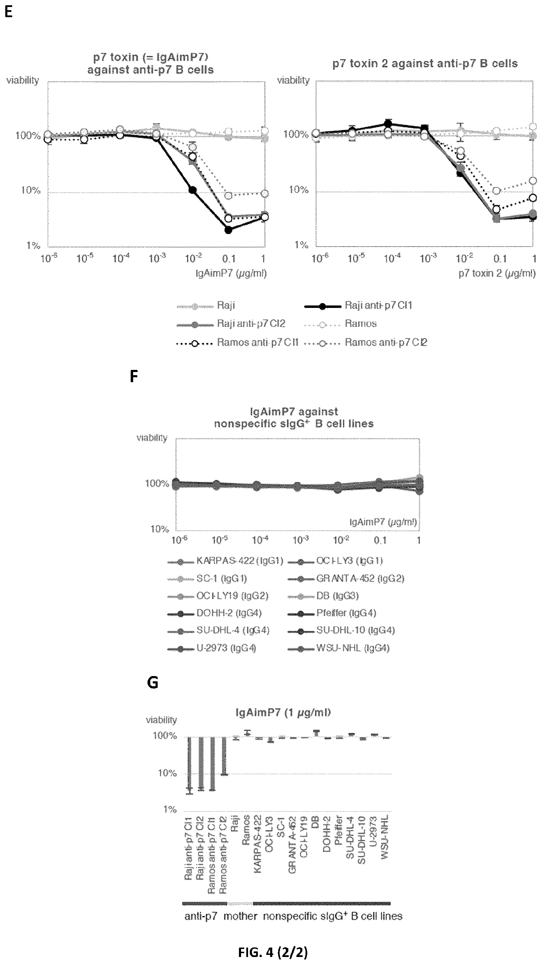

**Figure 4****. IgAimP7: anti-allergen B cell eraser. A.** A lentiviral vector to express human monoclonal antibody against the pollen allergen Phl p7. **B.** Overview of the strategy. The anti-Phl p7 monoclonal antibody expression vector was transfected into the slg- B cell line, Raji, and the sorted slgM- population of the Ramos B cell line. **C.** Design of two toxins using Phl p7. The p7 toxin was renamed to IgAimP7. **D.** Purification of the toxin proteins. Purified toxin proteins are highlighted by triangles. **E.** The cytotoxicity of p7 toxins against the B cell lines overexpressing anti-p7 IgG1 antibody. Experiments were performed in triplicate. **F.** The cytotoxicity of IgAimP7 against nonspecific slgG⁺ B cell lines. **G.** Comparison of the cytotoxicity of IgAimP7 to specific and nonspecific slgG⁺ B cell lines.

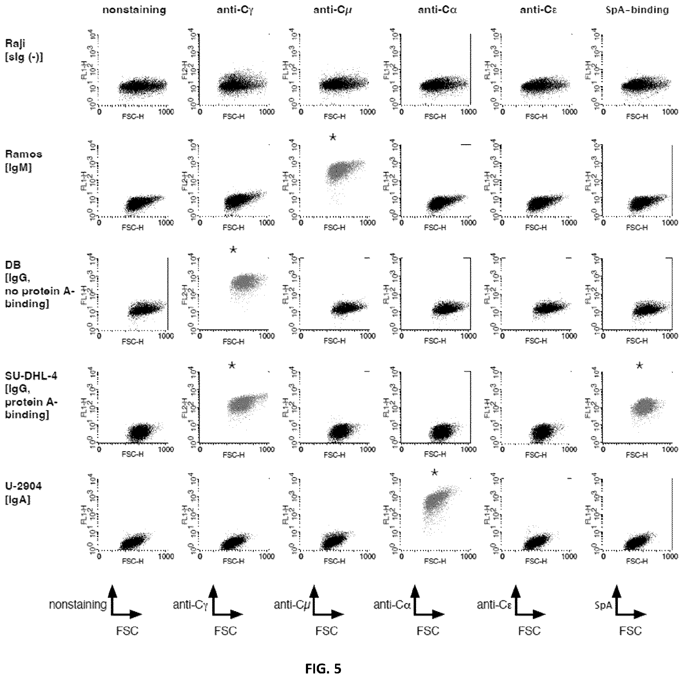

**Figure 5****. Evaluation of B cell classes by flow cytometry.** B cell classes were evaluated by antibody staining and SpA binding. The 30 B cells lines were classified into five categories: sIg⁻, sIgM⁺, sIgG⁺/SpA-bound, sIgG⁺/SpA-unbound, and slgA⁺. Of the 30 B cell lines analyzed, FACS profiles of representative cell lines in the five categories are shown. FACS profiles showing binding to the respective antibodies or SpA are highlighted with asterisks.

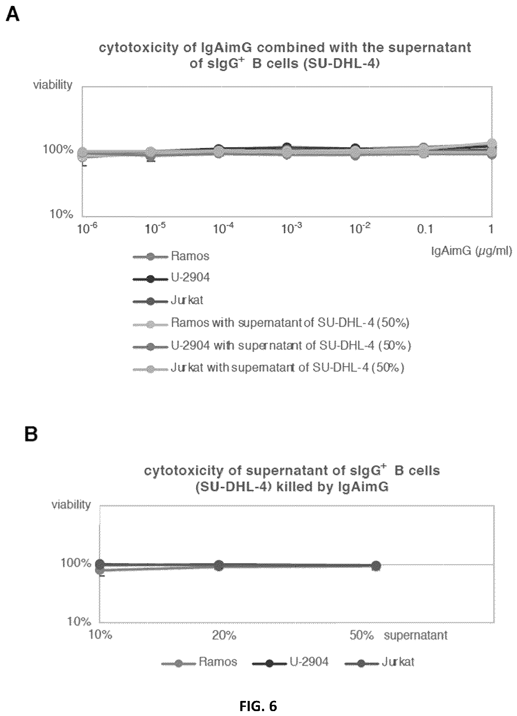

**Figure 6****. Culture supernatants of slgG⁺ cells do not show nonspecific cytotoxicity when combined with IgAimG. A.** slgG⁺ B cells such as SU-DHL-4 are efficiently killed by IgAimG. The sIgG⁺ cytotoxicity of IgAimG is most probably mediated by IgG on the B cell surface. Still, it was examined whether the complex of IgAimG and IgG secreted by slgG⁺ B cells is non-specifically cytotoxic. In the presence of 50% culture supernatant of slgG⁺ B cell line SU-DHL-4, IgAimG was not cytotoxic to the Ramos B cell line (sIgM⁺), U-2904 B cell line (sIgA⁺), or Jurkat T cell line. **B.** It was examined whether the contents released by IgAimG-killed B cells are cytotoxic to other cells. sIgG⁺ B cell line SU-DHL-4 was efficiently killed by three days of culture with 1 µg/ml of IgAimG. The culture medium containing 50% of this supernatant was not cytotoxic to the Ramos B cell line (sIgM⁺), the U-2904 B cell line (sIgA⁺), or the Jurkat T cell line.

### EXAMPLES

### Methods

### Design and construction of IgAim

The control vector was constructed by introducing amino acid substitutions of E82K and K179E into pET-22b DT 51E/148K (Addgene, #11081) to revert the inactive diphtheria toxin to the active form and by deleting amino acids 2-31 and 418-566, corresponding to the pel B signal peptide and the receptor binding domain, respectively. The Xhol site between the translocation domain and the histidine-tag (His-tag) of the resulting protein was used for cloning fusion partner proteins, specifically surface immunoglobulin (slg) B cell targeting peptide. The following proteins were used as fusion partners: SpA; SpA ZZ domain of pKK-TEV-ProteinA (*22*) (addgene #105788), SpG, amino acids 17-73 of pET His6 ProteinG TEV LIC cloning vector (1P) (Addgene, #29660), ZigA; mutations according to Gunneriusson et al. (*13*) were introduced in SpA sequence, Phl p7 full-length coding sequence of 78 amino acids (Y17835). The anti-Phl p7 human monoclonal antibody expression vector was constructed by introducing antibody light and heavy chain PCR product into a BOS-promoter lentiviral expression vector, where the antibody light chain gene and heavy chain VDJ gene are derived from pVITRO1-102.1F10-IgE/lambda (Addgene #50365), membrane-type Cgamma1 gene from KARPAS-422 cDNA, and a furin T2A linker from oligos (*16*). The GGGS linker (SSGGGSSGGGS (SEQ ID NO. 5)), the furin T2A linker (RRKRGSGLINEGRGSLLTCGDVEENPGP (SEQ ID NO. 25)), and the Phl p7 gene were created by PCR amplification of overlapping oligos. pET-22b DT 51E/148K is commercially available as Addgene plasmid # 11081; http://n2t.net/addgene:11081; RRID:Addgene_11081. pKK-TEV-ProteinA is commercially available as Addgene plasmid # 105788; http://n2t.net/addgene:105788; RRID:Addgene_105788. pET His6 ProteinG TEV LIC cloning vector (1P) is commercially available as Addgene plasmid # 29660; http://n2t.net/addgene:29660; RRID:Addgene_29660. pVlTRO1-102.1F10-IgE/λ is commercially available as Addgene plasmid # 50365; http://n2t.net/addgene:50365; RRID:Addgene_50365.

### Protein purification

IgAim expression vectors were transformed into T7 Express Competent E. coli (High Efficiency) (NEB, C2566H). Logarithmically growing bacteria were incubated with 0.4 mM IPTG for two h at 37°C to induce IgAim protein expression. E. coli were lysed by NEBExpress E. coli Lysis Reagent (NEB, P8116S), and His-tagged proteins were purified by NEBExpress Ni Spin Columns (NEB, S1427L). Protein concentrations were measured with Qubit Protein Assay Kit (Thermo Fischer, Q33211).

### Cell culture

The culture medium for B and other suspension cell lines is RPMI with 10% fetal bovine serum, 1% L-glutamine, 1% penicillin/streptomycin, and 0.05 mM 2- mercaptoethanol. The culture medium for adherent cell lines is DMEM with 10% fetal bovine serum, 1% L-glutamine, and 1% penicillin/streptomycin. Cell lines were cultured at 37°C with 5% CO2. Packaging and transduction of the anti-Phl p7 monoclonal antibody expression lentiviral vector were performed as previously described in (*23*). The vector was transfected into the slg- B cell line, Raji, and sorted the sig- population of the Ramos B cell line, and the clones highly expressing the Ig were selected by limiting dilution.

### Cell viability assay

Cells were cultured as a triplicate in 96 well multitier plates with different concentrations of IgAim. Suspension cell lines were cultured in 200 µl volumes, and adherent cell lines were cultured in 100 µl volumes. After about 70 hours of incubation with IgAim, 50 µl of suspension cells or 100 µl of adherent cells were incubated with 50 µl of CellTiter-Glo Luminescent Cell Viability Assay (Promega, G7571) at room temperature for 20 minutes and luminescence was measured on a Victor 3 plate reader (PerkinElmer).

### Flow cytometry

The following antibodies and SpA were used to test the antibody class of B cells: Goat anti-Human IgG (Fc specific)-Cy3 antibody (Merck, C2571-1ML), Goat anti-Human IgM (mu-chain specific)-FITC antibody (sigma, F5384-1ML), Goat Anti-Human IgA (alpha-chain specific)-FITC antibody (Sigma, F5259-1ML), Goat anti-Human IgE Fc Secondary Antibody (FITC) (Invitrogen, #H15701), Protein A-FITC Conjugate (Thermo Fisher, 101011). Flow cytometry was performed on a FACS Calibur (Becton Dickinson).

### Results

### Example 1

### IgAim: cell surface Ig-Aimed Immune Memory erasers

Each B cell can be distinguished by the antigen specificity or the class of its antibodies (Figure 1A). Figure 1B shows the expected mechanism of action of antibody-binding toxins derived from diphtheria toxin. After binding to the antibody, the artificial toxin is translocated to endosomes by receptor-mediated endocytosis. The acidic pH of the endosome causes a conformational change in the transition domain and membrane, allowing the release of DtA into the cytoplasm (*6*). The strategy for erasing immune memory by selectively removing B cells is summarized in Figure 1C. Diphtheria toxin fusion genes are cloned into an E. coli expression vector under the control of the T7 promoter, followed by transformation into T7 Express E. coli. After the fusion protein expression was induced by IPTG, these toxins containing a histidine (His) tag were purified using Ni-NTA spin columns. Cell lines were cultured in the presence of the engineered toxin proteins for three days, and cell viability was measured (Figure 1C).

SpA exhibits binding affinity to all IgG subclasses, except for IgG3 (*6*), whereas SpG can bind to all subclasses of human IgG antibodies (*7*). In this study, one control toxin and four toxins to target slgG⁺ B cells were designed (Figure 1D). A GGGS linker was inserted between the diphtheria toxin catalytic domain and SpA/SpG in toxins SpA toxin 2 and SpG toxin 2. These artificial toxins were identified as additional bands after IPTG induction and were purified using Ni-NTA spin columns (Figure 1E).

The cytotoxicity of these proteins was assessed in the slgG⁺ B cell line SU-DHL-4 (Figure 1F). Control toxins did not exhibit any toxicity. The mechanism of diphtheria toxin cytotoxicity involves translocating the toxic domain to the cytoplasm through a conformational change in the translocation domain at acidic pH. If the toxin domain is nonspecifically taken up by cells via endocytosis, it may remain in the endosomes without resulting in toxicity (*4*). The SpG-toxin showed weak cytotoxicity at a 1 µg/ml concentration. In contrast, both SpA-toxin and SpA-toxin 2 showed significant toxicity at concentrations as low as 0.0001 µg/ml and effectively killed 99% of cells above 0.001 µg/ml. Consequently, the SpA-toxin 2 was renamed IgAimG and investigated further to evaluate its toxicity against other slgG⁺ B cell lines.

### Example 2

### IgAimG: sIgG⁺ B cell eraser

Antibody classes and SpA binding were evaluated in a panel of 30 B cell lines (Figure 6), identifying 12 of sIgG⁺ B cell lines. Subclasses of slgG⁺ B cell lines were further identified by partial sequencing of their Cγ cDNA. It has previously been reported that the serum levels of four IgG subclasses, IgG1, IgG2, IgG3, and IgG4, correspond to approximately 67%, 22%, 7%, and 4% of total IgG levels, respectively (*1*). While IgG4 is typically the least abundant subclass in physiological conditions, it is noteworthy that several IgG4-expressing B-cell lines were present, suggesting a possible pathogenic relationship between B lymphoma with chronic inflammation due to IgG4-related diseases (*10*).

The slgG⁺ B-cell lines were subjected to a three-day culture in the presence of IgAimG. IgAimG showed potent cytotoxicity to all the slgG⁺ B cell lines except for the IgG3 B cell line DB. The resistance of DB to IgAimG is likely due to the inability of SpA to bind IgG3 (*6*). Indeed, despite its high levels of slgG, DB did not bind to SpA (Figure 5). Figure 2B plots the slgG levels of each slgG⁺ B-cell line evaluated by flow cytometry against IgAimG sensitivity, revealing a partial correlation between slgG expression levels and IgAimG sensitivity. IgG4 B cells (diamonds in Figure 2B) were relatively more sensitive to IgAimG. Interestingly, cells with low levels of slgG expression (U-2973, Pfeiffer, OCI-LY19) were also susceptible to killing by IgAimG (Figure 2B). It is plausible that not only the level of slgG expression but also the efficiency of slgG endocytosis plays a role in its efficacy (*11*).

IgAimG did not show toxicity to sIgG⁻ B-cell lines, namely slg-, sIgM⁺, and slgA⁺ B-cell lines (Figure 2C). Furthermore, IgAimG did not show toxicity towards non-B cell lines derived from various organs (Figure 2D). Figure 2E summarizes the results of Figures 2A, C, and D; IgAimG did not exert toxicity on non-IgG-expressing cells even at high concentrations (1 µg/ml), whereas it efficiently killed slgG⁺ B cells at low concentrations (0.01 µg/ml). Additionally, neither IgAimG combined with the supernatant from slgG⁺ B cells nor the supernatant from IgAimG-killed cells (Figure 6) exhibited nonspecific toxicity (Figure 6). Thus, the toxicity of IgAimG appears to be specific to slgG⁺ B cells of IgG1, G2, and G4 subclasses.

### EXAMPLE 3

### IgAimA: slgA⁺ B cell eraser

Replacement of 13 surface residues of SpA can change its binding specificity to different target proteins (*12*). Proteins made by randomization of 13 amino acids of SpA and screened by altered binding specificities are called affibodies (*12*). Thus, affibodies, similar to antibodies, are engineered to possess diverse binding specificities. Among these affibodies, ZigA is specifically designed to bind exclusively to IgA and not IgG (*13*).

The Inventor substituted the receptor binding domain of the diphtheria toxin with a monomer or a tandem repeat of ZigA, which can specifically bind human Cα (Figure 3A). These artificial toxin proteins were purified, as shown in Figure 3B. ZigA toxin showed toxicity to a sIgA⁺ B cell line, U-2904, at a concentration of 0.1 µg/ml and killed nearly 99% of the cells at 1 µg/ml (Figure 3C left). The toxic activity was further enhanced by the tandem ZigA in ZigA toxin 2, which displayed toxicity at a concentration of 0.001 µg/ml, effectively killing nearly 99% of IgA⁺ B cells at a concentration of 0.01 µg/ml (Figure 3C right). The ZigA toxin 2 was therefore renamed to IgAimA. Notably, the toxicity of IgAimA was specific to IgA⁺ B cell line. Other B-cell classes and non B-cell lines were unaffected by IgAimA (Figure 3C-E).

### EXAMPLE 4

### IgAimP7: anti-allergen B cell eraser

To selectively eliminate antigen-specific B cells, the receptor binding domain of diphtheria toxin was replaced by an antigen. A human antibody gene (*14*) specific for Phl p7 (*15*), a common allergen causing pollinosis, was cloned into a lentiviral vector and expressed under the BOS promoter, as illustrated in Figure 4A. The vector was designed to enable post-translational separation of the Ig light and heavy chains through a linker containing two cleavage sites of furin and T2A (*16*). The antibody expression vector was subsequently introduced into slg- Raji B cells or sorted slgM-Ramos B cells (Figure 4B). Single and tandem variants of the Phl p7-toxin were designed (Figure 4C), and proteins purified (Figure 4D). Both p7 toxin and p7 toxin 2 displayed toxicity against anti-p7 Raji and anti-p7 Ramos cells at concentrations of 0.01 µg/ml, effectively killing over 90% of anti-p7 B cell lines at 0.1 µg/ml (Figure 4E). The cytotoxic effects were similar for both p7 toxin variants; the toxin containing single Phl P7 (lgAimP7) is sufficient to kill anti-p7 B cells (Figure 4E, left). Importantly, they did not exhibit toxicity towards their mother cell lines or nonspecific slgG⁺ B cell lines (Figure 4F).

### Discussion

In this work, the Inventor developed a class of artificial toxins, termed IgAim, that can be customized and specifically targeted to the antibodies on the surface of B cells. IgAim can be tailored to eliminate monoclonal B cell populations by targeting the antigen-binding site, or IgAim can be employed more broadly to eliminate entire classes of B cells.

The IgAim control toxin was not toxic to the slgG⁺ B cell line (Figure 1E). IgAimG was toxic only to slgG⁺ B cells of the IgG1, IgG2, and IgG4 subclasses (Figure 2A), and IgAimA was toxic only to slgA⁺ B cells (Figure 3C). Importantly, none of these IgAims were toxic to other B cells or non-B cells (Figure 2A, 2C-E, 3C-D).

IgAimP7 was toxic to B cells expressing anti-Phl p7 antibodies but not to their parental population (Figure 4E).

Thus, the toxicity of IgAim depends on binding to the cell surface antibodies.

Furthermore, IgAimG was not toxic to the macrophage cell line THP-1 (Figure 2D). Moreover, the supernatant of slgG⁺ cells killed by IgAimG did not have nonspecific toxicity (Figure 6). This suggests that the stochastic uptake of IgAim via endocytosis is insufficient to induce cytotoxicity.

These data suggest that IgAim could be a highly effective therapy with a minimal risk for off-target effects.

IgAim is considered to have low nonspecific toxicity in vivo, as diphtheria toxin does not harm mice that do not naturally have diphtheria toxin receptors (5).

One application of IgAim is to eliminate extensive immune memory. Removing slgG⁺ B cells can effectively erase the overall adverse immune "trauma" associated with B cells. It is important to note that IgAimG does not kill IgG3⁺ B cells. However, IgG1, IgG2, and IgG4 collectively constitute approximately 93% of the total IgG in the bloodstream (1). Targeting these three IgG subclasses could significantly mitigate the harmful immune response by slgG⁺ B cells.

This study's B cell lines killed by IgAimG are derived from B leukemia and lymphoma (Figure 2A, 3C).

### SEQUENCES

The proteins mentioned above are preferably characterized by the following sequences:
IgAimG - AA sequence 
MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGL
SLTEPLMEQVGTEEFIKRFGDGASRVVLSLPFAEGSSSVEYINNWEQAKALSVELE
INFETRGKRGQDAMYEYMAQACAGNRVRRSVGSSLSCINLDWDVIRDKTKTKIES
LKEHGPIKNKMSESPNKTVSEEKAKQYLEEFHQTALEHPELSELKTVTGTNPVFA
GANYAAWAVNVAQVIDSETADNLEKTTAALSILPGIGSVMGIADGAVHHNTEEIVA
QSIALSSLMVAQAIPLVGELVDIGFAAYNFVESIINLFQVVHNSYNRPAYSPGHKTS
SGGGSSGGGSAVDNKFNKEQQNAFYEILHLPNLNEEQRNAFIQSLKDDPSQSANLL
AEAKKLNDAQAPKVDNKFNKEQQNAFYEILHLPNLNEEQRNAFIQSLKDDPSQSAN
LLAEAKKLNGAQAPKVDANSAGKSTLEHHHHHH (SEQ ID NO. 1)
**diphtheria toxin without receptor binding domain** 
MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKT (SEQ ID
NO. 4)
GGGS linker
   SSGGGSSGGGSA (SEQ ID NO. 5)
ZZ *domain (SpA)* 
VDNKFNKEQQNAFYEILHLPNLNEEQRNAFIQSLKDDPSQSANLLAEAKKLNDAQAP
KVDNKFNKEQQNAFYEILHLPNLNEEQRNAFIQSLKDDPSQSANLLAEAKKLNGAQ
APK (SEQ ID NO. 6)
His-tag
   HHHHHH (SEQ ID NO. 7)
IgAimG - DNA sequence 
ATGGGCGCTGATGATGTTGTTGATTCTTCTAAATCTTTTGTGATGGAAAACTTT
TCTTCGTACCACGGGACTAAACCTGGTTATGTAGATTCCATTCAAAAAGGTAT
 
ACAAAAGCCAAAATCTGGTACACAAGGAAATTATGACGATGATTGGAAAGGG
TTTTATAGTACCGACAATAAATACGACGCTGCGGGATACTCTGTAGATAATGA
AAACCCGCTCTCTGGAAAAGCTGGAGGCGTGGTCAAAGTGACGTATCCAGGA
CTGACGAAGGTTCTCGCACTAAAAGTGGATAATGCCGAAACTATTAAGAAAGA
GTTAGGTTTAAGTCTCACTGAACCGTTGATGGAGCAAGTCGGAACGGAAGAG
TTTATCAAAAGGTTCGGTGATGGTGCTTCGCGTGTAGTGCTCAGCCTTCCCTTC
GCTGAGGGGAGTTCTAGCGTTGAATATATTAATAACTGGGAACAGGCGAAAG
CGTTAAGCGTAGAACTTGAGATTAATTTTGAAACCCGTGGAAAACGTGGCCAA
GATGCGATGTATGAGTATATGGCTCAAGCCTGTGCAGGAAATCGTGTCAGGC
GATCAGTAGGTAGCTCATTGTCATGCATAAATCTTGATTGGGATGTCATAAGG
GATAAAACTAAGACAAAGATAGAGTCTTTGAAAGAGCATGGCCCTATCAAAAA
TAAAATGAGCGAAAGTCCCAATAAAACAGTATCTGAGGAAAAAGCTAAACAA
TACCTAGAAGAATTTCATCAAACGGCATTAGAGCATCCTGAATTGTCAGAACT
TAAAACCGTTACTGGGACCAATCCTGTATTCGCTGGGGCTAACTATGCGGCGT
GGGCAGTAAACGTTGCGCAAGTTATCGATAGCGAAACAGCTGATAATTTGGA
AAAGACAACTGCTGCTCTTTCGATACTTCCTGGTATCGGTAGCGTAATGGGCA
TTGCAGACGGTGCCGTTCACCACAATACAGAAGAGATAGTGGCACAATCAAT
AGCTTTATCGTCTTTAATGGTTGCTCAAGCTATTCCATTGGTAGGAGAGCTAGT
TGATATTGGTTTCGCTGCATATAATTTTGTAGAGAGTATTATCAATTTATTTCAA
GTAGTTCATAATTCGTATAATCGTCCCGCGTATTCTCCGGGGCATAAAACGTCT
AGCGGAGGTGGCTCTAGCGGTGGAGGATCCGCAGTGGACAACAAATTCAACAA
AGAACAACAAAACGCGTTCTATGAGATCTTACATTTACCTAACTTAAACGAAGAA
CAACGAAACGCCTTCATCCAAAGTTTAAAAGATGACCCAAGCCAAAGCGCTAAC
CTTTTAGCAGAAGCTAAAAAGCTAAATGATGCTCAGGCGCCGAAAGTAGACAAC
AAATTCAACAAAGAACAACAAAACGCGTTCTATGAGATCTTACATTTACCTAACTT
AAACGAAGAACAACGAAACGCCTTCATCCAAAGTTTAAAAGATGACCCAAGCCA
AAGCGCTAACCTTTTAGCAGAAGCTAAAAAGCTAAATGGTGCTCAGGCGCCGAA
AGTAGACGCGAATTCCGCGGGGAAGTCAACCCTCGAGCACCACCACCACCACC
ACTGA (SEQ ID NO. 8)
**diphtheria toxin without receptor binding domain** 
ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
GGTTCTCGCACTAAAAGTGGATAATGCCGAAACTATTAAGAAAGAGTTAGGTTTA
 
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA
GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACG (SEQ ID NO. 9)
GGGS linker
   TCTAGCGGAGGTGGCTCTAGCGGTGGAGGATCCGCA (SEQ ID NO. 10)
ZZ *domain (SpA)* 
GTGGACAACAAATTCAACAAAGAACAACAAAACGCGTTCTATGAGATCTTACATT
TACCTAACTTAAACGAAGAACAACGAAACGCCTTCATCCAAAGTTTAAAAGATGA
CCCAAGCCAAAGCGCTAACCTTTTAGCAGAAGCTAAAAAGCTAAATGATGCTCA
GGCGCCGAAAGTAGACAACAAATTCAACAAAGAACAACAAAACGCGTTCTATGA
GATCTTACATTTACCTAACTTAAACGAAGAACAACGAAACGCCTTCATCCAAAGT
TTAAAAGATGACCCAAGCCAAAGCGCTAACCTTTTAGCAGAAGCTAAAAAGCTA
AATGGTGCTCAGGCGCCGAAA (SEQ ID NO. 11)
His-tag
   CACCACCACCACCACCAC (SEQ ID NO. 12)
IgAimA - AA sequence 
MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGL
SLTEPLMEQVGTEEFIKRFGDGASRVVLSLPFAEGSSSVEYINNWEQAKALSVELE
INFETRGKRGQDAMYEYMAQACAGNRVRRSVGSSLSCINLDWDVIRDKTKTKIES
 
LKEHGPIKNKMSESPNKTVSEEKAKQYLEEFHQTALEHPELSELKTVTGTNPVFA
GANYAAWAVNVAQVIDSETADNLEKTTAALSILPGIGSVMGIADGAVHHNTEEIVA
QSIALSSLMVAQAIPLVGELVDIGFAAYNFVESIINLFQVVHNSYNRPAYSPGHKTS
SGGGSSGGGSAVDNKFNKETIQASQEIRLLPNLNGRQKLAFIHSLLDDPSQSANLL
AEAKKLNDAQAPKVDNKFNKETIQASQEIRLLPNLNGRQKLAFIHSLLDDPSQSAN
LLAEAKKLNGAQAPKVDANSAGKSTLEHHHHHH (SEQ ID NO. 2)
**diphtheria toxin without receptor binding domain** 
MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKT (SEQ ID
NO. 4)
GGGS linker
   SSGGGSSGGGSA (SEQ ID NO. 5)
***ZigA-ZigA*** 
VDNKFNKETIQASQEIRLLPNLNGRQKLAFIHSLLDDPSQSANLLAEAKKLNDAQAPK
VDNKFNKETIQASQEIRLLPNLNGRQKLAFIHSLLDDPSQSANLLAEAKKLNGAQAP
(SEQ ID NO. 13)
His-tag
   HHHHHH (SEQ ID NO. 7)
IgAimA - DNA sequence 
ATGGGCGCTGATGATGTTGTTGATTCTTCTAAATCTTTTGTGATGGAAAACTTT
TCTTCGTACCACGGGACTAAACCTGGTTATGTAGATTCCATTCAAAAAGGTAT
ACAAAAGCCAAAATCTGGTACACAAGGAAATTATGACGATGATTGGAAAGGG
TTTTATAGTACCGACAATAAATACGACGCTGCGGGATACTCTGTAGATAATGA
AAACCCGCTCTCTGGAAAAGCTGGAGGCGTGGTCAAAGTGACGTATCCAGGA
CTGACGAAGGTTCTCGCACTAAAAGTGGATAATGCCGAAACTATTAAGAAAGA
GTTAGGTTTAAGTCTCACTGAACCGTTGATGGAGCAAGTCGGAACGGAAGAG
TTTATCAAAAGGTTCGGTGATGGTGCTTCGCGTGTAGTGCTCAGCCTTCCCTTC
GCTGAGGGGAGTTCTAGCGTTGAATATATTAATAACTGGGAACAGGCGAAAG
CGTTAAGCGTAGAACTTGAGATTAATTTTGAAACCCGTGGAAAACGTGGCCAA
GATGCGATGTATGAGTATATGGCTCAAGCCTGTGCAGGAAATCGTGTCAGGC
GATCAGTAGGTAGCTCATTGTCATGCATAAATCTTGATTGGGATGTCATAAGG
 
GATAAAACTAAGACAAAGATAGAGTCTTTGAAAGAGCATGGCCCTATCAAAAA
TAAAATGAGCGAAAGTCCCAATAAAACAGTATCTGAGGAAAAAGCTAAACAA
TACCTAGAAGAATTTCATCAAACGGCATTAGAGCATCCTGAATTGTCAGAACT
TAAAACCGTTACTGGGACCAATCCTGTATTCGCTGGGGCTAACTATGCGGCGT
GGGCAGTAAACGTTGCGCAAGTTATCGATAGCGAAACAGCTGATAATTTGGA
AAAGACAACTGCTGCTCTTTCGATACTTCCTGGTATCGGTAGCGTAATGGGCA
TTGCAGACGGTGCCGTTCACCACAATACAGAAGAGATAGTGGCACAATCAAT
AGCTTTATCGTCTTTAATGGTTGCTCAAGCTATTCCATTGGTAGGAGAGCTAGT
TGATATTGGTTTCGCTGCATATAATTTTGTAGAGAGTATTATCAATTTATTTCAA
GTAGTTCATAATTCGTATAATCGTCCCGCGTATTCTCCGGGGCATAAAACGTCT
AGCGGAGGTGGCTCTAGCGGTGGAGGATCCGCAGTGGACAACAAATTCAACA
AAGAAACCATCCAGGCGTCCCAGGAGATCCGTCTGTTACCTAACTTAAACGGT
CGTCAAAAACTGGCCTTCATCCACAGTTTACTGGATGACCCAAGCCAAAGCGC
TAACCTTTTAGCAGAAGCTAAAAAGCTAAATGATGCTCAGGCGCCGAAAGTCG
ACAACAAATTCAACAAAGAAACGATTCAGGCGTCTCAGGAGATCCGCCTGTTA
CCTAACTTAAACGGTCGCCAAAAACTGGCCTTCATCCATAGTTTACTGGATGA
CCCAAGCCAAAGCGCTAACCTTTTAGCAGAAGCTAAAAAGCTAAATGGTGCTC
AGGCGCCGAAAGTAGACGCGAATTCCGCGGGGAAGTCAACCCTCGAGCACCA
CCACCACCACCACTGA (SEQ ID NO. 14)
**diphtheria toxin without receptor binding domain** 
ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
GGTTCTCGCACTAAAAGTGGATAATGCCGAAACTATTAAGAAAGAGTTAGGTTTA
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA
GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
 
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACG (SEQ ID NO. 9)
GGGS linker
   TCTAGCGGAGGTGGCTCTAGCGGTGGAGGATCCGCA (SEQ ID NO. 10)
***ZigA-ZigA*** 
GTGGACAACAAATTCAACAAAGAAACCATCCAGGCGTCCCAGGAGATCCGTCTG
TTACCTAACTTAAACGGTCGTCAAAAACTGGCCTTCATCCACAGTTTACTGGATG
ACCCAAGCCAAAGCGCTAACCTTTTAGCAGAAGCTAAAAAGCTAAATGATGCTC
AGGCGCCGAAAGTCGACAACAAATTCAACAAAGAAACGATTCAGGCGTCTCAG
GAGATCCGCCTGTTACCTAACTTAAACGGTCGCCAAAAACTGGCCTTCATCCAT
AGTTTACTGGATGACCCAAGCCAAAGCGCTAACCTTTTAGCAGAAGCTAAAAAG
CTAAATGGTGCTCAGGCGCCGAAA (SEQ ID NO. 15)
His-tag
   CACCACCACCACCACCAC (SEQ ID NO. 12)
IgAimP7 - AA sequence 
MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGL
SLTEPLMEQVGTEEFIKRFGDGASRVVLSLPFAEGSSSVEYINNWEQAKALSVELE
INFETRGKRGQDAMYEYMAQACAGNRVRRSVGSSLSCINLDWDVIRDKTKTKIES
LKEHGPIKNKMSESPNKTVSEEKAKQYLEEFHQTALEHPELSELKTVTGTNPVFA
GANYAAWAVNVAQVIDSETADNLEKTTAALSILPGIGSVMGIADGAVHHNTEEIVA
QSIALSSLMVAQAIPLVGELVDIGFAAYNFVESIINLFQVVHNSYNRPAYSPGHKTS
SGGGSSGGGSAASMADDMERIFKRFDTNGDGKISLSELTDALRTLGSTSADEVQR
MMAEIDTDGDGFIDFNEFISFCNANPGLMKDVAKVFVDLEHHHHHH (SEQ ID NO.
3)
**diphtheria toxin without receptor binding domain** 
MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
 
SLMVAQAIPLVGELVDIGFAAYNFVESIINLFQVVHNSYNRPAYSPGHKT (SEQ ID
NO. 4)
GGGS linker
   SSGGGSSGGGSA (SEQ ID NO. 5)
***Phl p7*** 
MADDMERIFKRFDTNGDGKISLSELTDALRTLGSTSADEVQRMMAEIDTDGDGFIDF
NEFISFCNANPGLMKDVAKVF (SEQ ID NO. 16)
His-tag
   HHHHHH (SEQ ID NO. 7)
IgAimP7 - DNA sequence 
ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
GGTTCTCGCACTAAAAGTGGATAATGCCGAAACTATTAAGAAAGAGTTAGGTTTA
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA
GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACGTCTAGCGGAGGTGGCTCTAGCGGTGGAGGATCA
GCAGCTAGCATGGCGGACGACATGGAGAGGATCTTCAAGAGGTTCGACACGAA
CGGTGACGGGAAGATCTCTCTGTCGGAGCTGACGGACGCACTGCGGACGCTG
GGCTCGACGTCGGCGGACGAGGTGCAGCGCATGATGGCGGAGATCGACACCG
ACGGCGACGGCTTCATCGACTTCAACGAGTTCATCTCCTTCTGCAACGCCAACC
 
CTGGACTCATGAAGGACGTCGCCAAGGTCTTCGTCGACCTCGAGCACCACCAC
CACCACCACTGA (SEQ ID NO. 17)
**diphtheria toxin without receptor binding domain** 
ATGGGCGCTGATGATGTTGTTGATTCTTCTAAATCTTTTGTGATGGAAAACTTTT
CTTCGTACCACGGGACTAAACCTGGTTATGTAGATTCCATTCAAAAAGGTATACA
AAAGCCAAAATCTGGTACACAAGGAAATTATGACGATGATTGGAAAGGGTTTTAT
AGTACCGACAATAAATACGACGCTGCGGGATACTCTGTAGATAATGAAAACCCG
CTCTCTGGAAAAGCTGGAGGCGTGGTCAAAGTGACGTATCCAGGACTGACGAA
GGTTCTCGCACTAAAAGTGGATAATGCCGAAACTATTAAGAAAGAGTTAGGTTTA
AGTCTCACTGAACCGTTGATGGAGCAAGTCGGAACGGAAGAGTTTATCAAAAGG
TTCGGTGATGGTGCTTCGCGTGTAGTGCTCAGCCTTCCCTTCGCTGAGGGGAG
TTCTAGCGTTGAATATATTAATAACTGGGAACAGGCGAAAGCGTTAAGCGTAGA
ACTTGAGATTAATTTTGAAACCCGTGGAAAACGTGGCCAAGATGCGATGTATGA
GTATATGGCTCAAGCCTGTGCAGGAAATCGTGTCAGGCGATCAGTAGGTAGCT
CATTGTCATGCATAAATCTTGATTGGGATGTCATAAGGGATAAAACTAAGACAAA
GATAGAGTCTTTGAAAGAGCATGGCCCTATCAAAAATAAAATGAGCGAAAGTCC
CAATAAAACAGTATCTGAGGAAAAAGCTAAACAATACCTAGAAGAATTTCATCAA
ACGGCATTAGAGCATCCTGAATTGTCAGAACTTAAAACCGTTACTGGGACCAAT
CCTGTATTCGCTGGGGCTAACTATGCGGCGTGGGCAGTAAACGTTGCGCAAGT
TATCGATAGCGAAACAGCTGATAATTTGGAAAAGACAACTGCTGCTCTTTCGATA
CTTCCTGGTATCGGTAGCGTAATGGGCATTGCAGACGGTGCCGTTCACCACAAT
ACAGAAGAGATAGTGGCACAATCAATAGCTTTATCGTCTTTAATGGTTGCTCAAG
CTATTCCATTGGTAGGAGAGCTAGTTGATATTGGTTTCGCTGCATATAATTTTGT
AGAGAGTATTATCAATTTATTTCAAGTAGTTCATAATTCGTATAATCGTCCCGCGT
ATTCTCCGGGGCATAAAACG (SEQ ID NO. 9)
GGGS linker
   TCTAGCGGAGGTGGCTCTAGCGGTGGAGGATCAGCA (SEQ ID NO. 10)
***Phl p7*** 
ATGGCGGACGACATGGAGAGGATCTTCAAGAGGTTCGACACGAACGGTGACGG
GAAGATCTCTCTGTCGGAGCTGACGGACGCACTGCGGACGCTGGGCTCGACGT
CGGCGGACGAGGTGCAGCGCATGATGGCGGAGATCGACACCGACGGCGACG
GCTTCATCGACTTCAACGAGTTCATCTCCTTCTGCAACGCCAACCCTGGACTCA
TGAAGGACGTCGCCAAGGTCTTC (SEQ ID NO. 18)
His-tag
   CACCACCACCACCACCAC (SEQ ID NO. 12)

### BIBLIOGRAPHY

1. H. W. Schroeder, Jr., L. Cavacini, Structure and function of immunoglobulins. The Journal of allergy and clinical immunology 125, S41-52 (2010).
2. H. Arakawa, J. Hauschild, J. M. Buerstedde, Requirement of the activation-induced deaminase (AID) gene for immunoglobulin gene conversion. Science 295, 1301-1306 (2002).
3. M. Muramatsu et al., Class switch recombination and hypermutation require activation-induced cytidine deaminase (AID), a potential RNA editing enzyme. Cell 102, 553-563 (2000).
4. F. Shafiee, M. G. Aucoin, A. Jahanian-Najafabadi, Targeted Diphtheria Toxin-Based Therapy: A Review Article. Frontiers in microbiology 10, 2340 (2019).
5. M. Saito et al., Diphtheria toxin receptor-mediated conditional and targeted cell ablation in transgenic mice. Nature biotechnology 19, 746-750 (2001).
6. G. Rigi, S. Ghaedmohammadi, G. Ahmadian, A comprehensive review on staphylococcal protein A (SpA): Its production and applications. Biotechnology and applied biochemistry 66, 454-464 (2019).
7. J. P. Derrick, D. B. Wigley, Crystal structure of a streptococcal protein G domain bound to an Fab fragment. Nature 359, 752-754 (1992).
8. I. H. Madshus, H. Stenmark, K. Sandvig, S. Olsnes, Entry of diphtheria toxin-protein A chimeras into cells. The Journal of biological chemistry 266, 17446-17453 (1991).
9. Y. Mazor, I. Barnea, I. Keydar, I. Benhar, Antibody internalization studied using a novel IgG binding toxin fusion. Journal of immunological methods 321, 41-59 (2007).
10. C. A. Perugino, J. H. Stone, IgG4-related disease: an update on pathophysiology and implications for clinical care. Nature reviews. Rheumatology 16, 702-714 (2020).
11. P. Hou et al., B cell antigen receptor signaling and internalization are mutually exclusive events. PLoS Biol 4, e200 (2006).
12. K. Nord et al., Binding proteins selected from combinatorial libraries of an alpha-helical bacterial receptor domain. Nature biotechnology 15, 772-777 (1997).
13. E. Gunneriusson et al., Staphylococcal surface display of immunoglobulin A (IgA)- and IgE-specific in vitro-selected binding proteins (affibodies) based on Staphylococcus aureus protein A. Applied and environmental microbiology 65, 4134-4140 (1999).
14. T. S. Dodev et al., A tool kit for rapid cloning and expression of recombinant antibodies. Scientific reports 4, 5885 (2014).
15. V. Niederberger et al., Calcium-dependent immunoglobulin E recognition of the apo- and calcium-bound form of a cross-reactive two EF-hand timothy grass pollen allergen, Phl p 7. FASEB journal : official publication of the Federation of American Societies for Experimental Biology 13, 843-856 (1999).
16. J. Chng et al., Cleavage efficient 2A peptides for high level monoclonal antibody expression in CHO cells. mAbs 7, 403-412 (2015).
17. G. Varano et al., The B-cell receptor controls fitness of MYC-driven lymphoma cells via GSK3β inhibition. Nature 546, 302-306 (2017).
18. N. Eroshenko et al., Implications of antibody-dependent enhancement of infection for SARS-CoV-2 countermeasures. Nature biotechnology 38, 789-791 (2020).
19. A. Vatti et al., Original antigenic sin: A comprehensive review. Journal of autoimmunity 83, 12-21 (2017).
20. L. Wang, F. S. Wang, M. E. Gershwin, Human autoimmune diseases: a comprehensive update. Journal of internal medicine 278, 369-395 (2015).
21. S. Ståhl et al., Affibody Molecules in Biotechnological and Medical Applications. Trends in biotechnology 35, 691-712 (2017).
22. R. J. Szczesny et al., Versatile approach for functional analysis of human proteins and efficient stable cell line generation using FLP-mediated recombination system. PloS one 13, e0194887 (2018).
23. H. Arakawa, A method to convert mRNA into a gRNA library for CRISPR/Cas9 editing of any organism. Sci Adv 2, e1600699 (2016).
24. Dodev et al. (2014) A tool kit for rapid cloning and expression of recombinant antibodies. Sci Rep. 4: 5885.
25. Wellmann et al. (2005) The evolution of human anti-double-stranded DNA autoantibodies Proc Natl Acad Sci U S A 102: 9258-9263.
26. Kipps et al. (1989) Molecular characterization of a major autoantibody-associated cross-reactive idiotype in Sjogren's syndrome. J Immunol. 142: 4261-4268.

## Claims

1. A fusion protein or conjugate comprising or consisting of:
a) a Diphtheria toxin deprived of the receptor binding domain, or functional fragments or derivatives or a biologically active variant thereof; and
b) at least one surface immunoglobulin (slg) B-cell targeting peptide, or functional fragments or derivatives or a biologically active variant thereof.

2. The fusion protein or conjugate according to claim 1 wherein said surface immunoglobulin (slg) B-cell targeting peptide of b) is selected from the group consisting of: peptides targeting a B-cell surface immunoglobulin G, peptides targeting a B-cell surface immunoglobulin A, peptides targeting a B-cell surface immunoglobulin E, peptides targeting a B-cell surface immunoglobulin against an autoimmune antigen, and peptides targeting a B-cell surface immunoglobulin against DNA.

3. The fusion protein or conjugate according to claim 1 or 2 wherein said at least one surface immunoglobulin (slg) B cell targeting peptide of b) comprises or consists of at least one peptide selected from the group consisting of:
i. at least one peptide derived from Staphylococcal protein A, preferably said peptide comprising or consisting of a sequence having at least 80% of identity with the sequence of ZZ domain or ZigA domain of Staphylococcal protein A,
ii. at least one peptide derived from Streptococcal protein G, preferably said peptide comprising or consisting of a sequence having at least 80% of identity with the sequence of IgG binding domain of Streptococcal protein G, and
iii. at least one peptide derived from Phleum pratense p7 domain, preferably said peptide comprising or consisting of a sequence having at least 80% of identity with the sequence of Phleum pratense p7 domain,
preferably said peptide is selected from (i.) a peptide comprising or consisting of at least one Staphylococcal protein A ZZ domain, (ii.) a peptide comprising or consisting of at least one, preferably two, Staphylococcal protein A ZigA domains (ZigA-ZigA) and a peptide comprising or consisting of at least one Phleum pratense p7 domain.

4. The fusion protein or conjugate according to any one of previous claims 1 to 3, further comprising a linker, said linker being preferably selected from the group of:
GGGS linker, Proline-rich linker, and (Thr-Gly-Ser)n linker wherein n is 1, 2, 3, 4, 5, 6, 7, 8, 9, 10, 11, 12, 13, 14, 15, 16, 17, 18, 19, or 20, said linker being more preferably GGGS linker; and/or
further comprising at least one peptide tag, said peptide tag being preferably selected from the group of:
histidine tag, Glutathione S-transferase (GST) tag, Maltose-binding protein (MBP) tag, S tag, FLAG tag, and Hemagglutinin (HA) tag, more preferably being histidine tag.

5. The fusion protein or conjugate according to any one of previous claims, wherein:
a) said diphtheria toxin has at least 80% sequence identity with, or comprises or consists of a sequence of SEQ ID NO. 4; preferably it comprises or consists of a sequence of SEQ ID NO. 4, and/or
b) said linker has at least 80% sequence identity with, or comprises or consists of a sequence of SEQ ID NO. 5, preferably it comprises or consists of a sequence of SEQ ID NO. 5; and/or
c) said surface immunoglobulin (slg) B cell targeting peptide has at least 80% sequence identity with, or comprises or consists of a sequence selected from the group consisting of:
i. a sequence of SEQ ID NO. 6,
ii. a sequence of SEQ ID NO. 13, and
iii. a sequence of SEQ ID NO. 16,
and/or
d) said peptide tag has at least 80% sequence identity with, or comprises or consists of a sequence of SEQ ID NO. 7, preferably it comprises or consists of a sequence of SEQ ID NO: 7,
preferably said fusion protein or conjugate comprises from N-terminus to C-terminus said components a), b), c) and d).

6. The fusion protein or conjugate according to any one of previous claims being able to target at least one surface immunoglobulin positive (slg+) B cell, preferably targeting IgG+B-cells or IgA+B-cells or IgE+B-cells, or Phleum pratense p7 expressing B-cells, or B-cells expressing surface antibodies against DNA.

7. The fusion protein or conjugate according to any one of previous claims, comprising from N-terminus to C-terminus:
i. a Diphtheria toxin deprived of the receptor binding domain comprising or consisting of a sequence having at least 80% sequence identity with, or comprising or consisting of a sequence of SEQ ID NO: 4 or functional fragments or derivatives or a biologically active variant thereof; and
ii. a GGGS linker comprising or consisting of a sequence having at least 80% sequence identity with, or comprising or consisting of a sequence of SEQ ID NO: 5; and
iii. a Staphylococcal protein A ZZ domain comprising or consisting of a sequence having at least 80% sequence identity with, or comprising or consisting of a sequence of SEQ ID NO: 6 or functional fragments or derivatives or a biologically active variant thereof; and
iv. a histidine tag comprising or consisting of a sequence having at least 80% sequence identity with, or comprising or consisting of a sequence of SEQ ID NO: 7;
or
i. a Diphtheria toxin deprived of the receptor binding domain comprising or consisting of a sequence having at least 80% sequence identity with, or comprising or consisting of a sequence of SEQ ID NO: 4 or functional fragments or derivatives or a biologically active variant thereof; and
ii. a GGGS linker comprising or consisting of a sequence having at least 80% sequence identity with, or comprising or consisting of a sequence of SEQ ID NO: 5; and
iii. two Staphylococcal protein A ZigA (ZigA-ZigA) domains comprising or consisting of a sequence having at least 80% sequence identity with, or comprising or consisting of a sequence of SEQ ID NO: 13 or functional fragments or derivatives or a biologically active variant thereof; and
iv. a histidine tag comprising or consisting of a sequence having at least 80% sequence identity with, or comprising or consisting of a sequence of SEQ ID NO: 7;
or
i. a Diphtheria toxin deprived of the receptor binding domain comprising or consisting of a sequence having at least 80% sequence identity with, or comprising or consisting of a sequence of SEQ ID NO: 4 or functional fragments or derivatives or a biologically active variant thereof; and
ii. a GGGS linker comprising or consisting of a sequence having at least 80% sequence identity with, or comprising or consisting of a sequence of SEQ ID NO: 5; and
iii. a Phleum pratense p7 domain comprising or consisting of a sequence having at least 80% sequence identity with, or comprising or consisting of a sequence of SEQ ID NO: 16 or functional fragments or derivatives or a biologically active variant thereof; and
iv. a histidine tag comprising or consisting of a sequence having at least 80% sequence identity with, or comprising or consisting of a sequence of SEQ ID NO: 7.

8. The fusion protein or conjugate according to any one of previous claims having at least 80% sequence identity, preferably at least 90% sequence identity, with, or comprising or consisting of a sequence of SEQ ID NO: 1 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAVDNKFNKEQQNAFYEILHLPNLNEEQRNAFIQSLKDDPSQSANLLAEAKKL
NDAQAPKVDNKFNKEQQNAFYEILHLPNLNEEQRNAFIQSLKDDPSQSANLLAEAK
KLNGAQAPKVDANSAGKSTLEHHHHHH),
 or of a sequence of SEQ ID NO: 2 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAVDNKFNKETIQASQEIRLLPNLNGRQKLAFIHSLLDDPSQSANLLAEAKKLN
DAQAPKVDNKFNKETIQASQEIRLLPNLNGRQKLAFIHSLLDDPSQSANLLAEAKKLN
GAQAPKVDANSAGKSTLEHHHHHH),
 or of a sequence of SEQ ID NO: 3 
(MGADDVVDSSKSFVMENFSSYHGTKPGYVDSIQKGIQKPKSGTQGNYDDDWKGF
YSTDNKYDAAGYSVDNENPLSGKAGGVVKVTYPGLTKVLALKVDNAETIKKELGLSL
TEPLMEQVGTEEFIKRFGDGASRVVLSLPFAEGSSSVEYINNWEQAKALSVELEINF
 
ETRGKRGQDAMYEYMAQACAGNRVRRSVGSSLSCINLDWDVIRDKTKTKIESLKE
HGPIKNKMSESPNKTVSEEKAKQYLEEFHQTALEHPELSELKTVTGTNPVFAGANY
AAWAVNVAQVIDSETADNLEKTTAALSILPGIGSVMGIADGAVHHNTEEIVAQSIALS
SLMVAQAIPLVGELVDIGFAAYNFVESIINLFQVVHNSYNRPAYSPGHKTSSGGGSS
GGGSAASMADDMERIFKRFDTNGDGKISLSELTDALRTLGSTSADEVQRMMAEIDT
DGDGFIDFNEFISFCNANPGLMKDVAKVFVDLEHHHHHH),
 or functional fragments, equivalents, variants, mutants, derivatives, synthetics or recombinants functional analogues thereof.

9. An isolated nucleic acid encoding the fusion protein or conjugate according to any one of claims 1-8, preferably said nucleic acid having at least 80% sequence identity, preferably at least 90% sequence identity, with or comprising or consisting of a sequence of SEQ ID NO. 8, SEQ ID NO. 14 or SEQ ID NO. 17.

10. A recombinant expression vector comprising the isolated nucleic acid of claim 9, preferably said isolated nucleic acid being operably linked to at least one regulatory sequence capable of directing expression of a gene encoding said fusion protein or conjugate in a host, or a host cell comprising and/or expressing the fusion protein or conjugate of claims 1-8, or the isolated nucleic acid of claim 9 or said recombinant expression vector comprising the isolated nucleic acid of claim 9, said host cell preferably being selected from the group consisting of: fungal cells, bacterial cells, preferably gram negative bacteria such as Escherichia coli, gram-positive Bacilli such as B. subtilis, B. licheniformis, B. megaterium, B. amyloliquefaciens, B. pumilus, plant protoplasts, unicellular algae, actinomycetales such as Streptomyces sp., and yeasts, such as Saccharomyces cerevisiae, Pichia pastoris, Yarrowia lipolytica, most preferably Trichoderma reesei or Bacillus, most preferably T7 Express Competent Escherichia coli.

11. A method of producing the fusion protein or conjugate of any one of claims 1-8 comprising the steps of transforming a host cell with an expression vector encoding said fusion protein, culturing said host cell under conditions enabling expression of said fusion protein, and optionally recovering and purifying said fusion protein.

12. A composition comprising the fusion protein or conjugate of any one of claims 1-8, or a pharmaceutical composition comprising the fusion protein or conjugate of any one of claims 1-8 and at least one pharmaceutically acceptable carrier.

13. The fusion protein or conjugate of any one of claims 1-8, the isolated nucleic acid of claim 9, the recombinant expression vector or the host cell of claim 10, or the pharmaceutical composition of claim 12 for use as a medicament.

14. The fusion protein or conjugate of any one of claims 1-8, the isolated nucleic acid of claim 9, the recombinant expression vector or the host cell of claim 10, or the pharmaceutical composition claim 12 for use in the treatment of a disease related to a disfunction or a misfunctioning of B-cells or of a sub-population of B-cells.

15. The fusion protein or conjugate or the isolated nucleic acid or the recombinant expression vector or the host cell or the pharmaceutical composition for the use of claim 14 wherein said disease is selected from the group consisting of: autoimmune diseases, allergies, B-cell dependent leukemia, nephropathy, Henoch-Schönlein purpura, celiac disease, and dermatologic herpetiformis, preferably said autoimmune disease being an autoimmune disease of musculoskeletal system, digestive system, nervous system, endocrine system, or skin, such as rheumatoid arthritis (RA), systemic lupus erythematosus (lupus), scleroderma, Sjögren's syndrome, dermatomyositis, ankylosing spondylitis, psoriatic arthritis, reactive arthritis, polymyalgia rheumatica, celiac disease, inflammatory bowel disease (IBD), Crohn's disease, ulcerative colitis, autoimmune hepatitis, primary sclerosing cholangitis, multiple sclerosis (MS), Guillain-Barré syndrome, myasthenia gravis, type 1 diabetes, Hashimoto's thyroiditis, Graves' disease, Addison's disease, vitiligo, alopecia areata, psoriasis, Raynaud's disease, vasculitis, sarcoidosis, autoimmune hemolytic anemia, or idiopathic thrombocytopenic purpura (ITP).
